(19) Europäisches Patentamt
European Patent Office
Office européen des brevets

(11) Veröffentlichungsnummer: **0 362 135 A2**

(12) **EUROPÄISCHE PATENTANMELDUNG**

(21) Anmeldenummer: **89810708.1**

(51) Int. Cl.5: **C07D 239/42 , A01N 43/54**

(22) Anmeldetag: **19.09.89**

(30) Priorität: **28.09.88 CH 3595/88**

(43) Veröffentlichungstag der Anmeldung:
**04.04.90 Patentblatt 90/14**

(84) Benannte Vertragsstaaten:
**AT BE CH DE ES FR GB GR IT LI LU NL**

(71) Anmelder: **CIBA-GEIGY AG**
**Klybeckstrasse 141**
**CH-4002 Basel(CH)**

(72) Erfinder: **Riebli, Peter**
**Bünten 17**
**CH-4446 Buckten(CH)**

(54) **Schädlingsbekämpfungsmittel.**

(57) Verbindungen der Formel

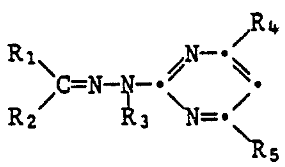

(I)

in welcher bedeuten:

$R_1$ Phenyl, Naphthyl, 1- bis 5-fach mit $R_6$ substituiertes Phenyl oder 1-bis 5-fach mit $R_6$ substituiertes Naphthyl;

$R_2$ $C_1$-$C_6$-Alkyl, mindestens einfach mit Halogen, $C_1$-$C_3$-Alkoxy und/oder $C_2$-$C_6$-Cycloalkyl substituiertes $C_1$-$C_6$-Alkyl, $C_3$-$C_6$-Cycloalkyl, $C_1$-$C_4$-Alkoxycarbonyl, Phenyl, 1- bis 3-fach mit Halogen, $C_1$-$C_3$-Alkoxy und/oder $C_1$-$C_3$-Alkyl substituiertes Phenyl, Benzyl oder 1- bis 3-fach mit Halogen, $C_1$-$C_3$-Alkoxy und/oder $C_1$-$C_3$-Alkyl substituiertes Benzyl;

$R_3$ Wasserstoff, $C_1$-$C_6$-Alkyl oder den Rest CO-$R_7$;

$R_4$ $C_3$-$C_6$-Cycloalkyl, 1- bis 3-fach mit Halogen und/oder Methyl substituiertes $C_3$-$C_6$-Cycloalkyl, mindestens einfach mit Halogen, Hydroxy und/oder Cyano substituiertes $C_1$-$C_4$-Alkyl, $C_2$-$C_5$-Alkinyl, $C_1$-$C_4$-Alkylthio oder den Rest $CH_2XR_8$;

$R_5$ Wasserstoff, $C_1$-$C_6$-Alkyl, mindestens einfach mit Halogen, Hydroxy, Cyano und/oder $C_1$-$C_3$-Alkoxy substituiertes $C_1$-$C_6$-Alkyl, $R_5$ ferner $C_3$-$C_5$-Alkenyl, Halogen, Hydroxy, $C_1$-$C_3$-Alkoxy, $C_1$-$C_3$-Haloalkoxy, $C_3$-$C_6$-Cycloalkyl oder $C_1$-$C_3$-Alkylthio;

$R_6$ unabhängig voneinander $C_1$-$C_6$-Alkyl, mit $C_1$-$C_3$-Alkoxy substituiertes $C_1$-$C_3$-Alkyl, $C_1$-$C_4$-Alkoxy, $C_1$-$C_3$-Haloalkyl, $C_1$-$C_3$-Haloalkoxy, Halogen, Cyano, Nitro, Hydroxy oder den Rest $S(O)_n$-$C_1$-$C_4$-Alkyl;

$R_7$ $C_1$-$C_6$-Alkyl oder ein mindestens einfach mit Halogen und/oder $C_1$-$C_3$-Alkoxy substituiertes $C_1$-$C_6$-Alkyl;

$R_8$ $C_1$-$C_6$-Alkyl, mindestens einfach mit Halogen, Hydroxy, Cyano und/oder $C_1$-$C_4$-Alkoxy substituiertes $C_1$-$C_6$-Alkyl, $C_3$-$C_6$-Alkenyl, $C_3$-$C_6$-Alkinyl oder $C_3$-$C_6$-Cycloalkyl;

X Sauerstoff oder Schwefel und

n 0, 1 oder 2;

unter Einschluss ihrer Säureadditionsverbindungen und ihrer Metallsalzkomplexe; besitzen wertvolle mikrobizide Eigenschaften. Die neuen Wirkstoffe können im Pflanzenschutz zur Verhütung des Befalls von Kulturpflanzen durch phytopathogene Mikroorganismen und zur Bekämpfung dieser Schädlinge eingesetzt

EP 0 362 135 A2

werden.

## Schädlingsbekämpfungsmittel

Die vorliegende Erfindung betrifft neue 2-Hydrazino-pyrimidin-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von Schädlingen, vor allem von pflanzenschädigenden Mikroorganismen, vorzugsweise Pilzen.

Die erfindungsgemässen Verbindungen entsprechen der allgemeinen Formel I

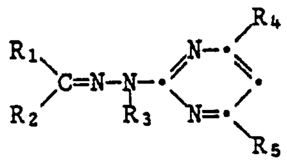

(I)

in welcher bedeuten:

$R_1$ Phenyl, Naphthyl, 1- bis 5-fach mit $R_6$ substituiertes Phenyl oder 1-bis 5-fach mit $R_6$ substituiertes Naphthyl;

$R_2$ $C_1$-$C_6$-Alkyl, mindestens einfach mit Halogen, $C_1$-$C_3$-Alkoxy und/oder $C_3$-$C_6$-Cycloalkyl substituiertes $C_1$-$C_6$-Alkyl, $C_3$-$C_6$-Cycloalkyl, $C_1$-$C_4$-Alkoxycarbonyl, Phenyl, 1- bis 3-fach mit Halogen, $C_1$-$C_3$-Alkoxy und/oder $C_1$-$C_3$-Alkyl substituiertes Phenyl, Benzyl oder 1- bis 3-fach mit Halogen, $C_1$-$C_3$-Alkoxy und/oder $C_1$-$C_3$-Alkyl substituiertes Benzyl;

$R_3$ Wasserstoff, $C_1$-$C_6$-Alkyl oder den Rest CO-$R_7$;

$R_4$ $C_3$-$C_6$-Cycloalkyl, 1- bis 3-fach mit Halogen und/oder Methyl substituiertes $C_3$-$C_6$-Cycloalkyl, mindestens einfach mit Halogen, Hydroxy und/oder Cyano substituiertes $C_1$-$C_4$-Alkyl, $C_2$-$C_5$-Alkinyl, $C_1$-$C_4$-Alkylthio oder den Rest $CH_2XR_8$;

$R_5$ Wasserstoff, $C_1$-$C_6$-Alkyl, mindestens einfach mit Halogen, Hydroxy, Cyano und/oder $C_1$-$C_3$-Alkoxy substituiertes $C_1$-$C_6$-Alkyl, $R_5$ ferner $C_3$-$C_5$-Alkenyl, Halogen, Hydroxy, $C_1$-$C_3$-Alkoxy, $C_1$-$C_3$-Haloalkoxy, $C_3$-$C_6$-Cycloalkyl oder $C_1$-$C_3$-Alkylthio;

$R_6$ unabhängig voneinander $C_1$-$C_6$-Alkyl, mit $C_1$-$C_3$-Alkoxy substituiertes $C_1$-$C_3$-Alkyl, $C_1$-$C_4$-Alkoxy, $C_1$-$C_3$-Haloalkyl, $C_1$-$C_3$-Haloalkoxy, Halogen, Cyano, Nitro, Hydroxy oder den Rest $S(O)_n$-$C_1$-$C_4$-Alkyl;

$R_7$ $C_1$-$C_6$-Alky oder ein mindestens einfach mit Halogen und/oder $C_1$-$C_3$-Alkoxy substituiertes $C_1$-$C_6$-Alkyl;

$R_8$ $C_1$-$C_6$-Alkyl, mindestens einfach mit Halogen, Hydroxy, Cyano und/oder $C_1$-$C_4$-Alkoxy substituiertes $C_1$-$C_6$-Alkyl, $C_3$-$C_6$-Alkenyl, $C_3$-$C_6$-Alkinyl oder $C_3$-$C_6$-Cycloalkyl;

X Sauerstoff oder Schwefel und

n 0, 1 oder 2;

unter Einschluss ihrer Säureadditionsverbindungen und ihrer Metallsalzkomplexe.

Unter Alkyl selbst oder als Bestandteil eines anderen Substituenten, wie Halogenalkyl, Alkoxy oder Halogenalkoxy, sind je nach Anzahl der angegebenen Kohlenstoffatome beispielsweise zu verstehen Methyl, Ethyl, Propyl, Butyl, Pentyl und Hexyl sowie ihre Isomeren, wie z.B. Isopropyl, Isobutyl, tert.-Butyl oder sek.-Butyl. Halogen, auch Hal genannt, steht für Fluor, Chlor, Brom oder Jod. Halogenalkyl und Halogenalkoxy bezeichnen einfach bis perhalogenierte Reste, wie z.B. $CHCl_2$, $CH_2F$, $CCl_3$, $CH_2Cl$, $CHF_2$, $CF_3$, $CH_2CH_2Br$, $C_2Cl_5$, CHBr, CHBrCl usw., vorzugsweise $CF_3$. Cycloalkyl steht je nach Zahl der genannten Kohlenstoffatome z.B. für Cyclopropyl, Cyclobutyl, Cyclopentyl oder Cyclohexyl.

Die Verbindungen der Formel I sind bei Raumtemperatur stabile Oele, Harze oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende fungizide Wirkung, sondern auch durch besonders gute Pflanzenverträglichkeit aus.

Die Erfindung betrifft sowohl die freien Verbindungen der Formel I als auch deren Additionssalze mit anorganischen und organischen Säuren sowie die Metallsalzkomplexe der Formel I.

Erfindungsgemässe Salze sind insbesondere Additionssalze mit unbedenklichen anorganischen Säuren, beispielsweise Halogenwasserstoffsäuren, z.B. Chlor-, Brom- oder Jodwasserstoffsäure oder wie Schwefelsäure, Phosphorsäure, phosphorige Säure, Salpetersäure, oder mit organischen Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salcylsäure, p-Aminosalicyclsäure, 2-Phenoxybenzoesäure, 2-Acetoxybenzoesäure oder 1,2-Naphthalin-disulfonsäure.

Erfindungsgemässe Metallsalzkomplexe der Formel I bestehen aus dem zugrundeliegenden organischen Molekül und einem anorganischen oder organischen Metallsalz, z.B. den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der zweiten Hauptgruppe wie Calcium und Magnesium und der dritten und vierten Hauptgruppe wie Aluminium, Zinn oder Blei sowie der ersten bis achten Nebengruppe wie Chrom, Mangan, Kobalt, Nickel, Kupfer, Zink usw. Bevorzugt sind die Nebengruppen-Elemente der 4. Periode. Die Metalle können dabei in den verschiedenen ihnen zukommenden Wertigkeiten vorliegen. Die Metallkomplexe können ein- oder mehrkernig auftreten, d.h. sie können ein oder mehrere organische Molekülanteile als Liganden enthalten.

Von den Verbindungen der Formel I existieren infolge der C = N-Doppelbindung zwei stereoisomere Formen, nämlich die E-Form und eine Z-Form. Im allgemeinen entsteht bei der Herstellung dieser Substanzen ein Gemisch beider Isomeren. Das Verhältnis, in dem sie gebildet werden, hängt von mehreren Bedingungen ab; so z.B. von der Struktur des Ausgangsmaterials, der Art des Reaktionsmediums, seines pH-Wertes, den Reaktionsbedingungen, wie Temperatur und Zeitraum und eventuell verwendeten Katalysator. Durch die Einstellung bestimmter Bedingungen kann ein einzelnes Isomeres in reiner Form synthetisiert werden. Wird ein Isomerengemisch erhalten, so können die reinen Komponenten auf verschiedene Art und Weise isoliert werden, so z.B. durch säulenchromatographische Trennungsmethoden. Die Identifizierung der Isomeren erfolgt üblicherweise durch NMR-spektro skopische Untersuchungen, wobei den Verbindungen der Formel I die "chemische Verschiebung" des Protonenresonanzsignals in der NH-Gruppe bestimmt wird. Die Verbindungen der Formel I können unter dem Einfluss von Wärme und Lichtstrahlung in ihre tautomeren Formen überführt werden.

Die vorliegende Erfindung umfasst hinsichtlich der Verbindungen der Formel I alle reinen Isomeren sowie deren Gemische.

Folgende Wirkstoffgruppen von Verbindungen der Formel I sind aufgrund ihrer ausgeprägten mikrobiziden, insbesondere pflanzenfungiziden, Aktivität bevorzugt:

Gruppe 1: Verbindungen der Formel I, worin bedeuten:

$R_1$ Phenyl, Naphthyl, bis zu 5-fach mit $R_6$ substituierte Phenyl oder bis zu 5-fach mit $R_6$ substituiertes Naphthyl;

$R_2$ $C_1$-$C_6$-Alkyl, 1- bis 5-fach mit Halogen oder 1-bis 2-fach mit $C_1$-$C_3$-Alkoxy und/oder 1-fach mit $C_3$-$C_6$-Cycloalkyl substituiertes $C_1$-$C_6$-Alkyl, $C_3$-$C_6$-Cycloalkyl, Phenyl, 1- bis 3-fach mit Halogen, $C_1$-$C_3$-Alkoxy und/oder $C_1$-$C_3$-Alkyl substituiertes Phenyl, Benzyl oder 1-bis 3-fach mit Halogen, $C_1$-$C_3$-Alkoxy und/oder $C_1$-$C_3$-Alkyl substituiertes Benzyl;

$R_3$ Wasserstoff, $C_1$-$C_3$-Alkyl oder den Rest CO-$R_7$;

$R_4$ $C_3$-$C_6$-Cycloakyl, 1- bis 3-fach mit Halogen und/oder Methyl substituiertes $C_3$-$C_6$-Cycloalkyl, mindestens einfach mit Halogen, Hydroxy und/oder Cyano substituiertes $C_1$-$C_4$-Alkyl, $C_2$-$C_5$-Alkinyl, $C_1$-$C_4$-Alkylthio oder den Rest $CH_2XR_8$;

$R_5$ Wasserstoff, $C_1$-$C_6$-Alkyl, mindestens einfach mit Halogen, Hydroxy, Cyano und/oder $C_1$-$C_3$-Alkoxy substituiertes $C_1$-$C_6$-Alkyl, $R_5$ ferner $C_3$-$C_5$-Alkenyl, Halogen, Hydroxy, $C_1$-$C_3$-Alkoxy, $C_1$-$C_3$-Haloalkoxy, $C_3$-$C_6$-Cycloakyl oder $C_1$-$C_3$-Alkylthio;

$R_6$ unabhängig voneinander $C_1$-$C_4$-Alkyl, $C_1$-$C_3$-Alkoxy, $C_1$-$C_3$-Haloalkyl, $C_1$-$C_3$-Halogen, Nitro oder den Rest $S(O)_n$-$C_1$-$C_3$-Alkyl;

$R_7$ $C_1$-$C_6$-Alkyl oder mindestens einfach mit Halogen und/oder $C_1$-$C_3$-Alkoxy substituiertes $C_1$-$C_6$-Alkyl;

$R_8$ $C_1$-$C_6$-Alkyl, mindestens einfach mit Halogen, Hydroxy und/oder $C_1$-$C_4$-Alkoxy substituiertes $C_1$-$C_6$-Alkyl, $C_3$-$C_6$-Alkenyl, $C_3$-$C_6$-Alkinyl oder $C_3$-$C_6$-Cycloalkyl;

X Sauerstoff oder Schwefel und

n 0, 1 oder 2.

Gruppe 2: Verbindungen der Formel I, worin bedeuten:

$R_1$ Phenyl, Naphthyl, bis zu 5-fach mit $R_6$ substituiertes Phenyl oder bis zu 3-fach mit $R_6$ substituiertes Naphthyl;

$R_2$ $C_1$-$C_6$-Alkyl, 1- bis 5-fach mit Halogen oder 1- bis 2-fach mit $C_1$-$C_3$-Alkoxy und/oder 1-fach mit $C_3$-$C_6$-Cycloalkyl substituiertes $C_1$-$C_6$-Alkyl, $C_3$-$C_6$-Cycloalkyl, Phenyl, 1- bis 3-fach mit Halogen, $C_1$-$C_3$-Alkoxy und/oder $C_1$-$C_3$-Alkyl substituiertes Phenyl, Benzyl oder 1-bis 3-fach mit Halogen, $C_1$-$C_3$-Alkoxy und/oder $C_1$-$C_3$-Alkyl substituiertes Benzyl;

$R_3$ Wasserstoff, $C_1$-$C_3$-Alkyl oder den Rest CO-$R_7$;

$R_4$ $C_3$-$C_6$-Cycloalkyl, 1- bis 3-fach mit Halogen und/oder Methyl substituiertes $C_3$-$C_6$-Cycloalkyl, $C_2$-$C_5$-

Alkinyl, $C_1$-$C_4$-Alkylthio oder den Rest $CH_2XR_8$;
$R_5$ Wasserstoff, $C_1$-$C_6$-Alkyl, mindestens einfach mit Halogen, Hydroxy, Cyano und/oder $C_1$-$C_3$-Alkoxy substituiertes $C_1$-$C_6$-Alkyl, $R_5$ ferner $C_3$-$C_5$-Alkenyl, Halogen, Hydroxy, $C_1$-$C_3$-Alkoxy, $C_1$-$C_3$-Haloalkoxy, $C_3$-$C_6$-Cycloalkyl oder $C_1$-$C_3$-Alkylthio;
$R_6$ unabhängig voneinander $C_1$-$C_4$-Alkyl, $C_1$-$C_3$-Alkoxy, $C_1$-$C_3$-Haloalkyl, $C_1$-$C_3$-Haloalkoxy, Halogen, Nitro oder den Rest $S(O)_n$-$C_1$-$C_3$-Alkyl;
$R_7$ $C_1$-$C_6$-Alkyl oder mindestens einfach mit Halogen und/oder $C_1$-$C_3$-Alkoxy substituiertes $C_1$-$C_6$-Alkyl;
$R_8$ $C_1$-$C_6$-Alkyl, mindestens einfach mit Halogen, Hydroxy und/oder $C_1$-$C_4$-Alkoxy substituiertes $C_1$-$C_6$-Alkyl, $C_3$-$C_6$-Alkenyl, $C_3$-$C_6$-Alkinyl oder $C_3$-$C_6$-Cycloalkyl;
X Sauerstoff oder Schwefel und
n 0, 1 oder 2.

Gruppe 3: Verbindungen der Formel I, worin bedeuten:
$R_1$ Phenyl, Naphthyl, bis zu 3-fach mit $R_6$ substituiertes Phenyl oder bis zu 3-fach mit $R_6$ substituiertes Naphthyl;
$R_2$ $C_1$-$C_6$-Alkyl, 1-fach mit $C_1$-$C_3$-Alkoxy oder $C_3$-$C_6$-Cycloakyl und/oder 1-bis 3-fach mit Halogen substituiertes $C_1$-$C_6$-Alkyl, $C_3$-$C_6$-Cycloakyl, Phenyl, 1- bis 3-fach mit Halogen und/oder $C_1$-$C_3$-Alkyl substituiertes Phenyl, Benzyl oder 1- bis 3-fach mit Halogen und/oder $C_1$-$C_3$-Alkyl substituiertes Benzyl;
$R_3$ Wasserstoff oder den Rest CO-$R_7$;
$R_4$ $C_3$-$C_6$-Cycloalkyl, 1- bis 3-fach mit Halogen und/oder Methyl substituiertes $C_3$-$C_6$-Cycloalkyl, $C_2$-$C_5$-Alkinyl, $C_1$-$C_4$-Alkylthio oder den Rest $CH_2XR_8$;
$R_5$ $C_1$-$C_6$-Alkyl, mit Hydroxy oder $C_1$-$C_3$-Alkoxy und/oder mindestens einfach mit Halogen substituiertes $C_1$-$C_6$-Alkyl, $R_5$ ferner $C_3$-$C_5$-Alkenyl, Halogen, Hydroxy, $C_1$-$C_3$-Alkoxy, $C_1$-$C_3$-Haloalkoxy, $C_3$-$C_6$-Cycloalkyl oder $C_1$-$C_3$-Alkylthio;
$R_6$ unabhängig voneinander $C_1$-$C_4$-Alkyl, $C_1$-$C_2$-Alkoxy, $C_1$-$C_2$-Haloalkoxy oder Halogen;
$R_7$ $C_1$-$C_4$-Alkyl oder mindestens einfach mit Halogen substituiertes $C_1$-$C_4$-Alkyl;
$R_8$ $C_1$-$C_6$-Alkyl oder mindestens einfach mit Halogen oder $C_1$-$C_4$-Alkoxy substituiertes $C_1$-$C_6$-Alkyl und
X Sauerstoff.

Gruppe 4: Verbindungen der Formel I, worin bedeuten:
$R_1$ bis zu 3-fach mit $R_6$ substituiertes Phenyl mit der Massgabe, dass mindestens die ortho-Stellung im Phenylring substituierte ist;
$R_2$ $C_1$-$C_3$-Alkyl;
$R_3$ Wasserstoff;
$R_4$ $C_3$-$C_6$-Cycloalkyl, mit $CH_3$ substituiertes $C_3$-$C_6$-Cycloalkyl, $C_2$-$C_4$-Alkinyl, $C_1$-$C_3$-Alkylthio oder den Rest $CH_2X$-$R_8$;
$R_5$ $C_1$-$C_3$-Alkyl, mit Halogen substituiertes $C_1$-$C_3$-Alkyl, $R_5$ ferner $C_3$-$C_5$-Alkenyl, $C_3$-$C_5$-Cycloalkyl oder $C_1$-$C_3$-Alkylthio;
$R_6$ unabhängig voneinander $C_1$-$C_4$-Alkyl, Halogen oder $C_1$-$C_4$-Haloalkyl;
$R_8$ $C_1$-$C_3$-Alkyl und
Z Sauerstoff.

Eine wichtige Gruppe von erfindungsgemässen pflanzenfungiziden Wirkstoffen sind diejenigen Verbindungen der Formel I, in denen die Phenylreste von $R_1$ und $R_2$ ortho-substituiert sind.

Gruppe 5: Verbindungen der Formel I, worin bedeuten:
$R_1$ α-Naphthyl, 2-substituiertes α-Naphthyl, 2,3-disubstituiertes oder 2,4-disubstituiertes α-Naphthyl, wobei die Substituenten in 2- und 4-Stellung $C_1$-$C_3$-Alkyl, Halogen oder Nitro sein können und der Substituent in 3-Stellung $C_1$-$C_3$-Alkyl ist;
$R_2$ $C_1$-$C_3$-Alkyl;
$R_3$ Wasserstoff;
$R_4$ $C_3$-$C_6$-Cycloalkyl, mit $CH_3$ substituiertes $C_3$-$C_6$-Cycloalkyl, $C_2$-$C_4$-Alkinyl, $C_1$-$C_3$-Alkylthio oder den Rest $CH_2X$-$R_8$;
$R_5$ $C_1$-$C_3$-Alkyl, mit Halogen substituiertes $C_1$-$C_3$-Alkyl, $R_5$ ferner $C_3$-$C_5$-Alkenyl, $C_3$-$C_5$-Cycloalkyl oder $C_1$-$C_3$-Alkylthio;
$R_8$ $C_1$-$C_3$-Alkyl und
X Sauerstoff.

Gruppe 6: Verbindungen der Formel I gemäss Gruppe 5, worin $R_1$ α-Naphthyl, 2-Methyl-α-naphthyl, 2-Methyl-4-nitro-α-naphthyl, 2,4-Dimethyl-α-naphthyl, 2,3-Dimethyl-α-naphthyl oder 4- Methyl-α-naphthyl bedeuten und die übrigen Substituenten die für Gruppe 5 genannte Bedeutung haben.

Als bevorzugte Verbindungen der Formel I sind folgende zu nennen:
4-Methyl-6-cyclopropyl-2-[1-(2-methylphenyl)-ethylidenhydrazino]-pyrimidin (Verb. Nr. 1.1);

4-Methyl-6-methoxymethyl-2-[1-(2-methylphenyl)-ethylidenhydrazino]-pyrimidin (Verb. Nr. 1.11);
4-Methyl-6-cyclopropyl-2-[1-(2-chlorphenyl)-ethylidenhydrazino]-pyrimidin (Verb. Nr. 1.13);
4-Methyl-6-(1-propinyl)-2-[1-(2-methylphenyl)-ethylidenhydrazino]-pyrimidin (Verb. Nr. 1.37);
4-Methyl-6-(1-propinyl)-2-[1-(2-chlorphenyl)-ethylidenhydrazino]-pyrimidin (Verb. Nr. 1.42);
4-Fluormethyl-6-cyclopropyl-2-[1-(2-methylphenyl)-ethylidenhydrazino]-pyrimidin (Verb. Nr. 1.53);
4-Methyl-6-fluormethyl-2-[1-(2-methylphenyl)-ethylidenhydrazino]-pyrimidin (Verb. Nr. 1.4);
4-Methyl-6-cyclopropyl-2-[1-(2,4-dimethylphenyl)-ethylidenhydrazino]-pyrimidin (Verb. Nr. 1.2);
4-Methyl-6-cyclopropyl-2-[1-(2-methyl-α-naphthyl)-ethylidenhydrazino]-pyrimidin (Verb. Nr. 3.25);
4-Methyl-6-cyclopropyl-2-[1-(2,4-dimethyl-α-naphthyl)-ethylidenhydrazino]-pyrimidin (Verb. Nr. 3.26);
4-Methyl-6-cyclopropyl-2-[1-(2,5-dimethylphenyl)-ethylidenhydrazino]-pyrimidin (Verb. Nr. 1.35);
4-Methyl-6-cyclopropyl-2-[1-(2-bromphenyl)-ethylidenhydrazino]-pyrimidin (Verb. Nr. 1.45);
4-Methyl-6-thiomethyl-2-[1-(2-methylphenyl)-ethylidenhydrazino]-pyrimidin (Verb. Nr. 1.168).

Die Verbindungen der Formel I werden hergestellt, indem man

1. ein aromatisches Keton der Formel II

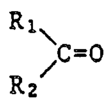

(II)

mit einem Hydrazinopyrimidin der Formel III

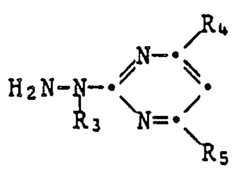

(III)

oder einem Salz davon in einem inerten Lösungsmittel oder in der Schmelze bei Temperaturen von -20 °C bis 200 °C, bevorzugt 20 °C bis zum Siedepunkt des verwendeten Lösungsmittels, umsetzt, wobei das Lösungsmittel auch das Keton der Formel II sein kann, und wobei ferner die Zugabe eines Katalysators in Form einer geringen Menge einer organischen oder anorganischen Säure oder einer Base eine beträchtliche Beschleunigung der Umsetzung bewirken kann; oder

2. ein Hydrazon der Formel IV

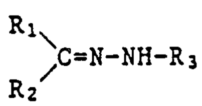

(IV)

mit einem Pyrimidin der Formel V

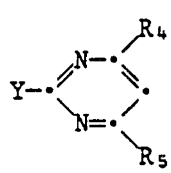

(V)

worin Y Halogen, bevorzugt Chlor oder Brom, $C_1$-$C_4$-Alkoxy, bevorzugt Methoxy oder Ethoxy, Phenoxy, Mercapto, $C_1$-$C_4$-Alkylthio, bevorzugt Methylthio oder Ethylthio, Phenylthio, $C_1$-$C_4$-Alkylsulfonyl, bevorzugt Methylsulfonyl oder Ethylsulfonyl, oder Phenylsulfonyl darstellt, in Gegenwart einer Base in einem inerten Lösungsmittel bei Temperaturen von -50 ° bis 150 °C, bevorzugt von 20 °C bis zum Siedepunkt des verwendeten Lösungsmittels, umsetzt; oder

3. ein Amidinohydrazon der Formel VI

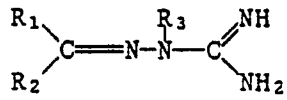

(VI)

mit einem Diketon der Formel VII

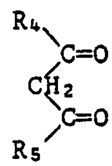

(VII)

in einem inerten Lösungsmittel oder in einer Schmelze bei Temperaturen von -20° bis 200° C, bevorzugt von 20° C bis zum Siedepunkt des verwendeten Lösungsmittels, umsetzt, wobei das Lösungsmittel auch das Diketon der Formel VII sein kann.

Darüber hinaus werden Verbindungen der Formel I, in denen $R_3$ $C_1$-$C_6$-Alkyl oder CO-$R_7$ darstellt und $R_7$ $C_1$-$C_6$-Alkyl oder ein mindestens einfach mit Halogen und/oder $C_1$-$C_3$-Alkoxy substituiertes $C_1$-$C_6$-Alkyl bedeutet, hergestellt, indem man
eine Verbindung der Formel Ia

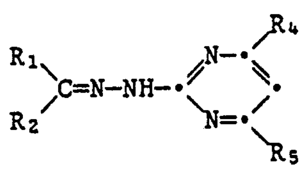

(Ia)

a) mit einer Verbindung der Formel VIII
$R_3$-Z (VIII)
worin Z eine übliche Abgangsgruppe, wie z.B. Halogen oder Sulfonyloxy, bedeutet, in Gegenwart einer starken Säure oder ohne diese in einem inerten Lösungsmittel umsetzt; oder

b) mit einem reaktionsfähigen Derivat einer Säure der Formel
$R_7$-COOH (IX),
wobei als reaktionsfähige Derivate von Verbindungen der Formel IX die Halogenide, bevorzugt Chloride oder Bromide, oder die Anhydride zu nennen sind.

Ferner werden Verbindungen der Formel Ic

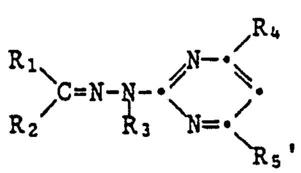

(Ic)

worin $R_5'$ Chlor oder Brom bedeutet, hergestellt, indem man eine Verbindung der Formel Ib

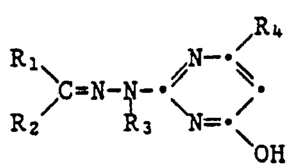

(Ib)

mit Phosphoroxychlorid oder Phosphoroxybromid in einem inerten Lösungsmittel bei Temperaturen von -20° bis 180° C, bevorzugt von 20° C bis zum Siedepunkt des verwendeten Lösungsmittels, umsetzt, wobei als Lösungsmittel auch das betreffende Phosphoroxyhalogenid eingesetzt werden kann.

Darüber hinaus werden Verbindungen der Formel Id

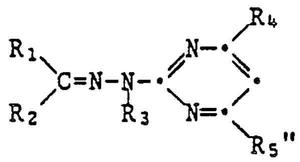

(Id)

worin $R_5''$ $C_1$-$C_3$-Alkoxy, $C_1$-$C_3$-Haloalkoxy oder $C_1$-$C_3$-Alkylthio bedeutet, hergestellt, indem man eine Verbindung der Formel Ic (vorstehend angegeben) mit einer Verbindung der Formel XI

$R^a$-X-M (XI)

worin $R^a$ $C_1$-$C_3$-Alkyl oder $C_1$-$C_3$-Haloalkyl, X Sauerstoff oder Schwefel und M Wasserstoff oder vorzugsweise ein Metallatom, insbesondere ein Alkaliatom, bedeuten, in einem inerten Lösungsmittel bei Temperaturen von -20°C bis 180°C, bevorzugt 20°C bis zum Siedepunkt des verwendeten Lösungsmittels reagieren lässt, wobei im Falle, dass M Wasserstoff darstellt, die Verbindung XI auch als Lösungsmittel eingesetzt werden kann.

In den vorhergehend beschriebenen Herstellungsverfahren stellen die Symbole $R_1$-$R_5$ die unter Formel I angegebenen Bedeutungen dar.

Die Verbindungen der Formel III werden hergestellt durch Umsetzung eines Hydrazinderivats der Formel X

$R_3$-NH-$NH_2$ (X)

mit einem Pyrimidinderivat der Formel V

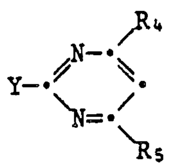

(V)

worin Y Halogen, bevorzugt Chlor oder Brom, $C_1$-$C_4$-Alkoxy, bevorzugt Methoxy oder Ethoxy, Phenoxy, Mercapto, $C_1$-$C_4$-Alkylthio, bevorzugt Methylthio oder Ethylthio, Phenylthio, $C_1$-$C_4$-Alkylsulfonyl, bevorzugt Methylsulfonyl oder Ethylsulfonyl, oder Phenylsulfonyl darstellt, in Gegenwart einer Base in einem inerten Lösungsmittel bei Temperaturen von -50° bis 150°C, bevorzugt von 20°C bis zum Siedepunkt des verwendeten Lösungsmittels (D.J. Brown, The Pyrimidines, Interscience Publishers, 1962; Bull. Soc. Chim. Belg. 68, 30, 1959; Chemical Pharmaceutical Bulletin 17, 1479, 1969; Australian J. Chem. 30, 2515, 1977).

Die Hydrazonderivate der Formel IV sind bekannt oder lassen sich nach bekannten Methoden herstellen, so z.B. durch Umsetzung eines Ketonderivates der Formel II mit einem Hydrazinderivat der Formel X [Eur. J. Med. Chem.-Chim. Ther. 12(5), 427, 1977; Org. Syn. 50, 102, 1970; J. Pharm. Sci. 61(10), 1571, 1972].

Die Pyrimidinderivate der Formel V sind teilweise bekannt oder lassen sich nach bekannten Methoden herstellen (D.J. Brown, The Pyrimidines, Interscience Publishers, 1962).

Die Amidinohydrazone der Formel VI sind teilweise bekannt oder lassen sich nach bekannten Methoden herstellen, so z.B. durch Umsetzung eines Ketonderivates der Formel II mit einem Salz eines Aminoguanidinderivates der Formel

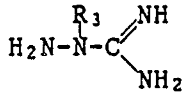

in Gegenwart einer Base (Liebigs Annalen der Chemie 307,293, 1899).

In den vorstehend beschriebenen Verfahren können in Anpassung an die jeweiligen Reaktionsbedingungen beispielsweise folgende Lösungsmittel verwendet werden:

Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Chlornaphthalin, Tetrachlorkohlenstoff, Trichlorethan, Trichlorethylen, Pentachlorethan, Difluorbenzol, 1,2-Dichlorethan, 1,1-Dichlorethan, 1,2-cis-Dichlorethylen, Chlorbenzol, Fluorbenzol, Brombenzol, Dichlorbenzol, Dibrombenzol, Chlortoluol, Trichlortoluol; Ether, wie Ethylpropylether, Methyl-tert.-butylether, n-Butylethylether, Di-n-butylether, Di-isobutylether, Diisoamylether, Diisopropyletether, Anisol, Cyclohexylmethylether, Diethylether, Ethylenglykoldimethylether,

Ethylenglykoldiethylether, Tetrahydrofuran, Dioxan, Dimethoxyethan, Thioanisol, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, wie Heptan, Hexan, Octan, Nonan, Cymol, Benzinfraktionen innerhalb eines Siedepunktintervalles von 70 °C bis 190 °C, Cyclohexan, Methylcyclohexan, Dekalin, Petrolether, Ligroin, Trimethylpentan, wie 2,3,3-Trimethylpentan; Ester wie Ethylacetat, Acetessigester, Isobutylacetat; Amide, z.B. Formamid, Methylformamid, Dimethylformamid; Ketone, wie Acetone, Methylethylketon; Alkohole, insbesondere niedere aliphatische Alkohole, wie z.B. Methanol, Ethanol, n-Propanol, iso-Propanol sowie die Isomeren der Butanole; und aromatische Kohlenwasserstoffe, wie Benzol, Toluol, Xylol. Auch Gemische der genannten Lösungs- und Verdünnungs-mittel kommen in Betracht.

Als Protonenakzeptoren z.B. anorganische oder organische Basen, wie beispielsweise Alkali- oder Erdalkaliverbindungen, z.B. die Hydroxide, Oxide oder Karbonate von Lithium, Natrium, Kalium, Magnesium, Calcium, Strontium und Barium oder auch Hydride wie z.B. Natriumhydrid. Als organische Basen seien beispielsweise tertiäre Amine wie Triethylamin, Triethylendiamin, Pyridin genannt.

Als Säuren können sowohl anorganische Säuren, wie z.B. Halogenwasserstoffsäuren, beispielsweise Fluorwasserstoffsäure, Chlorwasserstoffsäure oder Bromwasserstoffsäure, sowie Schwefelsäure, Phosphorsäure oder Salpetersäure als auch geeignete organische Säuren wie Essigsäure, Benzolsulfonsäure, Toluolsulfonsäure, Alkansäure, wie z.B. Methansäure u.a. verwendet werden.

Die in den Verbindungen der vorstehend beschriebenen Verfahren aufgeführten Reste $R_1$ bis $R_5$ besitzen die unter Formel I angegebenen Bedeutungen.

Die Verbindungen der Formel III sind neu und stellen einen Teil der vorliegenden Erfindung dar. Sie sind folgendermassen definiert:

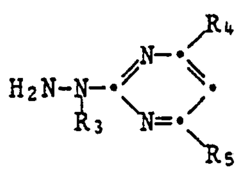

(III)

worin bedeuten:

$R_3$ Wasserstoff, $C_1$-$C_6$-Alkyl oder CO-$R_7$;

$R_4$ $C_3$-$C_6$-Cycloalkyl, bis zu 3-fach mit Halogen und/oder Methyl substituiertes $C_3$-$C_6$-Cycloalkyl, mindestens einfach mit Halogen, Hydroxy und/oder Cyano substituiertes $C_1$-$C_4$-Alkyl, $C_2$-$C_5$-Alkinyl, $C_1$-$C_4$-Alkylthio oder den Rest $CH_2XR_8$;

$R_5$ Wasserstoff, $C_1$-$C_6$-Alkyl, mindestens einfach mit Halogen, Hydroxy, Cyano und/oder $C_1$-$C_3$-Alkoxy substituiertes $C_1$-$C_6$-Alkyl, $R_5$ ferner $C_3$-$C_5$-Alkenyl, Halogen, $C_1$-$C_3$-Alkoxy, $C_1$-$C_3$-Haloalkoxy, $C_3$-$C_6$-Cycloalkyl oder $C_1$-$C_3$-Alkylthio;

$R^8$ $C_1$-$C_6$-Alkyl, mindestens einfach mit Halogen, Hydroxy, Cyano und/oder $C_1$-$C_4$-Alkoxy substituiertes $C_1$-$C_6$-Alkyl, $C_3$-$C_6$-Alkenyl, $C_3$-$C_6$-Alkinyl oder $C_3$-$C_6$-Cycloalkyl;

X Sauerstoff oder Schwefel.

Ueberraschenderweise wurde nun gefunden, dass Verbindungen der Formel I ein für praktische Bedürfnisse sehr günstiges biozides Spektrum zur Bekämpfung von phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. von phytopathogenen Mikroorganismen verschont bleiben.

Verbindungen der Formel I sind z.B. gegen die den folgenden Klassen angehörenden phytopathogenen Pilze wirksam: Fungi imperfecti (insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. Venturia und Erysiphe, Podosphaera, Monilinia, Uncinula) und der Oomyceten (z.B. Phytophthora, Pythium, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten,

insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch inniges Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spuren elemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk Blattaplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren ode verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha, bevorzugt 100 g bis 2 kg AS/ha, insbesondere bei 200 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglyukol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der

physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von granulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,9 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiel dienen zur näheren Erläuterung der Erfindung, ohne dieselbe einzuschränken.

## 1. Herstellungsbeispiele

Beispiele 1.1: Herstellung von 4-Methyl-6-cyclopropyl-2-[1-(2-methylphenyl)-ethylidenhydrazino]-pyrimidin

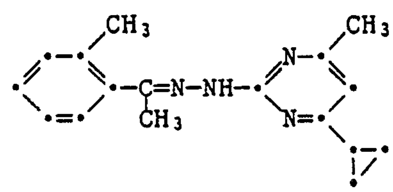

3,3 g 2-Hydrazino-4-methyl-6-cyclopropylpyrimidin und 2,7 g 2-Methylacetophenon werden in 40 ml Ethanol zum Sieden erhitzt und unter Rühren 20 Stunden unter Rückfluss gekocht. Anschliessend wird die Reaktionslösung abgekühlt und das Lösungsmittel unter Vakuum verdampft. Der ölige Rückstand wird zur Reinigung mittels Hexan/Ethylacetat (2:1) über eine Kieselgelsäule chromatographiert.

Dabei wird zuerst die E-Form, dann das E/Z-Gemisch und schliesslich die Z-Form der Titelverbindung eluiert.

Die verschiedenen Fraktionen werden einzeln isoliert.

E-From:

Schmelzpunkt: 121-122 °C

Z-Form:

Schmelzpunkt: 96-98 °C

E/Z Gemisch:

Schmelzpunkt: 92-93 °C

Die Zuordnung erfolgt mittels NMR Spektren.

Beispiel 1.2: Herstellung von 2-Hydrazino-4-methyl-6-cyclopropylpyrimidin

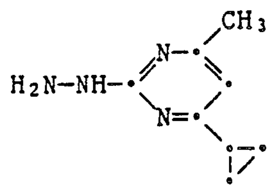

3,0 g 2-Chlor-4-methyl-6-cyclopropylpyrimidin werden in 30 ml Ethanol gelöst, 3,8 ml Hydrazinhydrat zugegeben und 20 Stunden bei Raumtemperatur gerührt. Anschliessend wird das Lösungsmitel unter Vakuum verdampft und der Rückstand mit Ethylacetat umkristallisiert. Smp. 98-99 °C.

Tabelle 1: Verbindungen der Formel

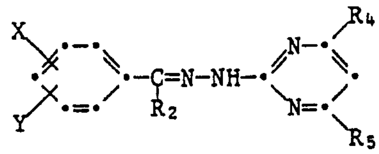

| Verb. Nr. | X | Y | $R_2$ | $R_4$ | $R_5$ | physikalische Konstante |
|---|---|---|---|---|---|---|
| 1.1 | 2-$CH_3$ | H | $CH_3$ | 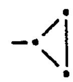 | $CH_3$ | Smp. 92-93°C |
| 1.2 | 2-$CH_3$ | 4-$CH_3$ | $CH_3$ | 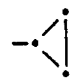 | $CH_3$ | Smp. 133-134°C |
| 1.3 | 3-Cl | H | $CH_3$ | 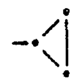 | $CH_3$ | Smp. 124-125°C |
| 1.4 | 2-$CH_3$ | H | $CH_3$ | $CH_2F$ | $CH_3$ | |
| 1.5 | 2-$CH_3$ | H | $CH_3$ | $CF_3$ | $CH_3$ | Smp. 125-135°C |
| 1.6 | 2-$CH_3$ | H | 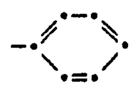 | $CH_2OCH_3$ | $CH_3$ | |
| 1.7 | 2-Cl | H | $CH_3$ | C≡CH | $CH_3$ | |
| 1.8 | 4-Cl | H | 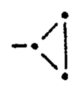 | 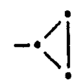 | $CH_3$ | Smp. 100-102°C |
| 1.9 | 2-F | H | $CH_3$ | 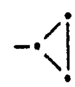 | $CH_3$ | Smp. 115-118°C |
| 1.10 | H | H | 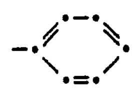 | 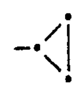 | $CH_3$ | Smp. 99-101°C |
| 1.11 | 2-$CH_3$ | H | $CH_3$ | $CH_2OCH_3$ | $CH_3$ | $n_D^{29}$: 1,5953 |
| 1.12 | 2-CN | H | $CH_3$ | $CF_3$ | $CH_3$ | |
| 1.13 | 2-Cl | H | $CH_3$ | 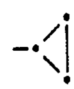 | $CH_3$ | Smp. 139-140°C |
| 1.14 | 2-$OCHF_2$ | 4-Cl | $CH_3$ | 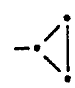 | $CH_3$ | |
| 1.15 | 2-Cl | 4-Cl | $CH_3$ | 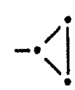 | $CH_3$ | |
| 1.16 | 2-$CH_3$ | 4-$CH_3$ | $CH_3$ | $CF_3$ | $CH_3$ | |
| 1.17 | 4-$CH_3$ | H | $CH_3$ | 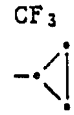 | $CH_3$ | Smp. 176-177°C |

Fortsetzung: Tabelle 1

| Verb. Nr. | X | Y | $R_2$ | $R_4$ | $R_5$ | physikalische Konstante |
|---|---|---|---|---|---|---|
| 1.18 | 2-Cl | H | 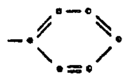 | 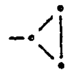 | $CH_3$ | Smp. 89-92°C |
| 1.19 | 2-$CH_3$ | 5-$CH_3$ | $CH_3$ | $CH_2OCH_3$ | $CH_3$ | |
| 1.20 | 2-$SCH_3$ | H | $CH_3$ | 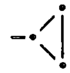 | $CH_3$ | |
| 1.21 | 2-$CH_3$ | 4-$CH_3$ | $CH_3$ | $CH_2F$ | $CH_3$ | |
| 1.22 | 2-$CH_3$ | H | $CH_3$ | 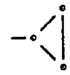 | $CH_2OCH_3$ | |
| 1.23 | 4-Cl | H | $CH_3$ | 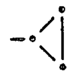 | $CH_3$ | Smp. 168-169°C |
| 1.24 | 2-$CH_3$ | H | $CH_3$ | 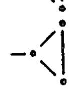 | 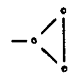 | |
| 1.25 | 3-F | H | $CH_3$ | 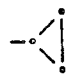 | $CH_3$ | |
| 1.26 | 2-$CH_3$ | 4-$CH_3$ | $CH_3$ | $CH_2OCH_3$ | $CH_3$ | |
| 1.27 | 2-$CH_3$ | H | $CH_3$ | 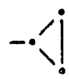 | $OCH_3$ | |
| 1.28 | 2-Br | H | $CH_3$ | $CF_3$ | $CH_3$ | |
| 1.29 | 2-$OCH_3$ | H | $CH_3$ | 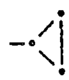 | $CH_3$ | Smp. 83-85°C |
| 1.30 | 2-$CH_3$ | 4-$OCHF_2$ | $CH_3$ | 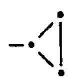 | $CH_3$ | $n_D^{52}$: 1,5672 |
| 1.31 | 2-$CH_3$ | H | $CH_3$ | $CH_2OCH_3$ | Cl | Smp. 116-117°C |
| 1.32 | H | H | $(CH_2)_3CH_3$ | 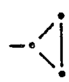 | $CH_3$ | |
| 1.33 | 2-$CH_3$ | H | 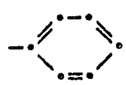 | 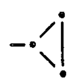 | $CH_3$ | $n_D^{52}$: 1,6258 |
| 1.34 | 2-Cl | H | $CH_3$ | $CF_3$ | $CH_3$ | |
| 1.35 | 2-$CH_3$ | 5-$CH_3$ | $CH_3$ | 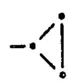 | $CH_3$ | Smp. 119-121°C |
| 1.36 | 2-$CH_3$ | H | $CH_2CH_3$ | 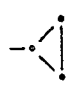 | $CH_3$ | |
| 1.37 | 2-$CH_3$ | H | $CH_3$ | $C{\equiv}C-CH_3$ | $CH_3$ | |
| 1.38 | H | H | $CH_3$ | 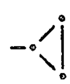 | $CH_3$ | Smp. 108-112°C |

Fortsetzung: Tabelle 1

| Verb. Nr. | X | Y | R2 | R4 | R5 | physikalische Konstante |
|---|---|---|---|---|---|---|
| 1.39 | 3-$CH_3$ | H | $CH_3$ | 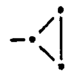 | $CH_3$ | Smp.121,5-122°C |
| 1.40 | 2-$CH_3$ | H | $CH_3$ | 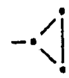 | Cl | |
| 1.41 | 2-$CF_3$ | H | $CH_3$ | 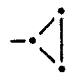 | $CH_3$ | Smp. 147-151°C |
| 1.42 | 2-Cl | H | $CH_3$ | C≡C-$CH_3$ | $CH_3$ | |
| 1.43 | 2-$CH_3$ | H | $CH_3$ | 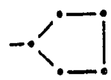 | $CH_3$ | |
| 1.44 | 2-$CH_3$ | H | $CH_3$ | $CH_2OCH_2CH_3$ | $CH_3$ | |
| 1.45 | 2-Br | H | $CH_3$ | 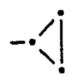 | $CH_3$ | Smp. 128-134°C |
| 1.46 | 2-$NO_2$ | H | $CH_3$ | 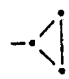 | $CH_3$ | Smp. 106-110°C |
| 1.47 | 2-$CH_3$ | 4-$OCHF_2$ | $CH_3$ | $CH_2OCH_3$ | $CH_3$ | |
| 1.48 | 2-$CH_3$ | 4-$CH_3$ | $CH_3$ | C≡C-$CH_3$ | $CH_3$ | |
| 1.49 | 2-$CH_3$ | H | $CH_3$ | 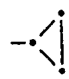 | $OCHF_2$ | |
| 1.50 | 2-$OCHF_2$ | H | $CH_3$ | 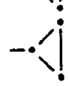 | $CH_3$ | Smp. 120-122°C |
| 1.51 | 2-$CH_3$ | H | $CH_3$ | $CH_2Cl$ | $CH_3$ | |
| 1.52 | 3-$CH_3$ | 4-$CH_3$ | $CH_3$ | 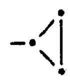 | $CH_3$ | Smp. 154-155°C |
| 1.53 | 2-$CH_3$ | H | $CH_3$ | 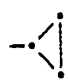 | $CH_2F$ | |
| 1.54 | 2-$CH_3$ | 5-$CH_3$ | $CH_3$ | $CH_2F$ | $CH_3$ | |
| 1.55 | 2-Cl | 5-Cl | $CH_3$ | 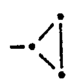 | $CH_3$ | |
| 1.56 | 2-$CH_3$ | 4-$CH_3$ | 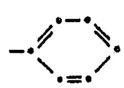 | 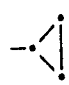 | $CH_3$ | |
| 1.57 | 2-$CH_3$ | 4-$OCHF_2$ | $CH_3$ | C≡C-$CH_3$ | $CH_3$ | |
| 1.58 | 2-Cl | H | 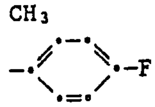 | 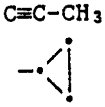 | $CH_3$ | |
| 1.59 | 2-$CH_3$ | H | 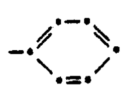 | $CH_2F$ | $CH_3$ | |

Fortsetzung: Tabelle 1

| Verb. Nr. | X | Y | $R_2$ | $R_4$ | $R_5$ | physikalische Konstante |
|---|---|---|---|---|---|---|
| 1.60 | 2-$CH_3$ | H | $CH_3$ | 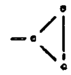 | $CF_3$ | |
| 1.61 | 2-$CH_3$ | 4-$CH_3$ | 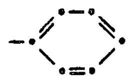 | $CH_2OCH_3$ | $CH_3$ | |
| 1.62 | 2-$CH_3$ | 4-Cl | $CH_3$ | 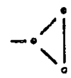 | $CH_3$ | |
| 1.63 | H | H | $CH_2$— 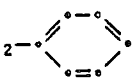 | 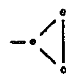 | $CH_3$ | $n_D^{54}$: 1,6290 |
| 1.64 | 2-$CH_3$ | H | $CH_3$ | $C{\equiv}CCH_2CH_2CH_3$ | $CH_3$ | |
| 1.65 | 2-Cl | H | $CH_3$ | 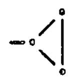 | $OCH_3$ | |
| 1.66 | 2-$CH_3$ | H | $CH_3$ | $CH_2OCH_3$ | $OCH_3$ | $n_D^{49}$: 1,5800 |
| 1.67 | 2-$CH_3$ | H | $CH_3$ | $C{\equiv}C-CH_3$ | Cl | |
| 1.68 | 2-$CH_3$ | H | $CH_3$ | 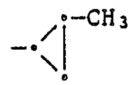 | $CH_2F$ | |
| 1.69 | 2-$CH_3$ | H | $CH_3$ | 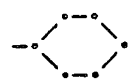 | $CH_3$ | |
| 1.70 | 2-Cl | H | $CH_3$ | $CH_2OCH_3$ | $CH_2CH_3$ | |
| 1.71 | 2-$CH_3$ | H | $CH_3$ | 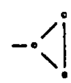 | $CH_2OH$ | |
| 1.72 | 2-$CH_3$ | H | $CH_3$ | 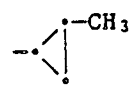 | $CH_3$ | |
| 1.73 | 2-Cl | H | $CH_3$ | $CH_2OCH_3$ | $CH_3$ | |
| 1.74 | 2-$CH_3$ | H | $CH_3$ | $C{\equiv}CH$ | $CH_3$ | |
| 1.75 | 2-$CH_3$ | 3-$CH_3$ | $CH_3$ | 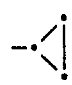 | $CH_3$ | |
| 1.76 | 2-$OCH_3$ | H | $CH_3$ | $CH_2OCH_3$ | $CH_3$ | |
| 1.77 | 2-$CH_3$ | 4-$OCH_3$ | $CH_3$ | 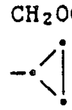 | $CH_3$ | Smp. 108-113°C |
| 1.78 | 2-Cl | H | $CH_3$ | 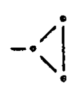 | $CH_2F$ | |
| 1.79 | 2-Br | H | $CH_3$ | $C{\equiv}C-CH_3$ | $CH_3$ | |
| 1.80 | H | H | $CH_2$— 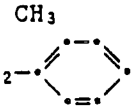 | $CH_2OCH_3$ | $CH_3$ | |
| 1.81 | 2-$CH_3$ | 4-$CH_3$ | 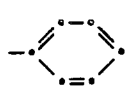 | 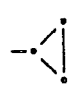 | $CH_2F$ | |

Fortsetzung: Tabelle 1

| Verb. Nr. | X | Y | $R_2$ | $R_4$ | $R_5$ | physikalische Konstante |
|---|---|---|---|---|---|---|
| 1.82 | 2-Cl | 4-Cl | $CH_3$ | $CH_2OCH_3$ | $CH_3$ | |
| 1.83 | 2-$CH_3$ | H | $CH_3$ | 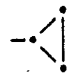 | $CH_2CH_3$ | |
| 1.84 | 2-$CH_3$ | H | $CH_3$ | $CHCl_2$ | $CH_3$ | |
| 1.85 | 2-$CH_3$ | H | $CH_3$ | $CF_2CF_3$ | $CH_3$ | |
| 1.86 | 2-$CH_3$ | H | $CH_3$ | $CH_2OCH_3$ | $CF_3$ | |
| 1.87 | 2-Cl | H | $CH_3$ | $CHCl_2$ | $CH_3$ | |
| 1.88 | 2-$CH_3$ | H | $CH_3$ | $CF_2Cl$ | $CH_3$ | |
| 1.89 | 2-$CH_3$ | H | $CH_3$ | $CH_2OCH_3$ | $CF_2Cl$ | |
| 1.90 | 2-$CH_3$ | H | $CH_3$ | $CH(CH_3)_2$ | $CF_3$ | |
| 1.91 | 2-Cl | H | $CH_3$ | $CF_2Cl$ | $CH_3$ | |
| 1.92 | 2-$CH_3$ | H | $CH_3$ | $(CH_2)_3CH_3$ | $CF_3$ | |
| 1.93 | 2-$CH_3$ | H | $CH_3$ | 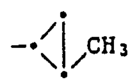 | 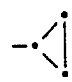 | |
| 1.94 | 2-$CH_3$ | H | $CH_3$ | $C\equiv C-CH_3$ | 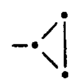 | |
| 1.95 | 2-$CH_3$ | 4-$CH_3$ | $CH_3$ | 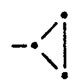 | $CH_2CH_3$ | |
| 1.96 | 2-Br | H | $CH_3$ | $CH_2OCH_3$ | $CH_3$ | |
| 1.97 | 2-F | H | $CH_3$ | $CH_2F$ | 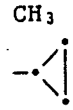 | |
| 1.98 | 2-$CH_3$ | H | $CH_3$ | $CH_2F$ | $CH_2F$ | |
| 1.99 | 2-$CH_3$ | 4-$OCH_3$ | $CH_3$ | $CH_2F$ | 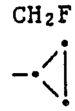 | |
| 1.100 | 2-$CH_3$ | 4-$OCH_3$ | $CH_3$ | $C\equiv C-CH_3$ | $CH_3$ | |
| 1.101 | 2-$CH_3$ | 5-$CH_3$ | $CH_3$ | $C\equiv C-CH_3$ | $CH_3$ | |
| 1.102 | 2-$CH_3$ | H | $CF_3$ | 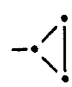 | $CH_3$ | |
| 1.103 | H | H | $CO_2CH_2CH_3$ | 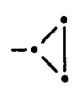 | $CH_3$ | |
| 1.104 | 2-$CH_3$ | 5-$CH_3$ | $CH_3$ | $CH_2F$ | 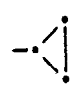 | |
| 1.105 | 2-$CH_3$ | H | $CH_2$ 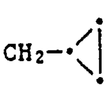 | 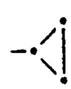 | $CH_3$ | |

Fortsetzung: Tabelle 1

| Verb. Nr. | X | Y | $R_2$ | $R_4$ | $R_5$ | physikalische Konstante |
|---|---|---|---|---|---|---|
| 1.106 | 2-$CH_3$ | H | $CH_2OCH_3$ | 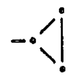 | $CH_3$ | |
| 1.107 | 2-$CH_3$ | H | 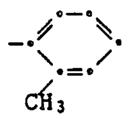 | 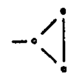 | $CH_3$ | |
| 1.108 | 2-$CH_3$ | H | 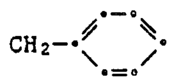 | 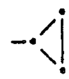 | $CH_3$ | |
| 1.109 | 2-$CH_3$ | H | 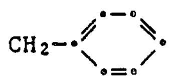 | $CH_2OCH_3$ | $CH_3$ | |
| 1.110 | 2-$CH_3$ | H | 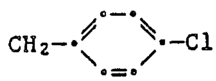 | 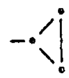 | $CH_3$ | |
| 1.111 | 2-$CH_3$ | H | $CH_3$ | $CH_2CN$ | 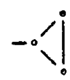 | |
| 1.112 | 2-Cl | H | $CH_3$ | $CH_2OH$ | 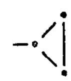 | |
| 1.113 | 2-$CH_3$ | H | $CH_3$ | $CH_2Br$ | 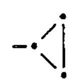 | |
| 1.114 | 2-$CH_3$ | H | $CH_3$ | $CF_3$ | $CH(OCH_3)_2$ | |
| 1.115 | 2-$CH_3$ | H | $CH_3$ | $CH_2OH$ | 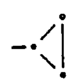 | |
| 1.116 | 2-$CH_3$ | H | $CH_2CH_3$ | $C\equiv C-CH_3$ | $CH_3$ | |
| 1.117 | 2-$CH_3$ | 4-$CH_3$ | $CH_2CH_3$ | $CH_2OCH_3$ | $CH_3$ | |
| 1.118 | 2-F | H | $CH_3$ | $C\equiv C-CH_3$ | $CH_3$ | |
| 1.119 | 2-$CH_3$ | H | 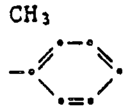 | 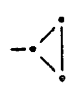 | $CH_2F$ | |
| 1.120 | 2-Cl | H | 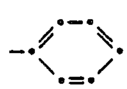 | $CH_2OCH_3$ | $CH_3$ | |
| 1.121 | 2-$CH_3$ | H | 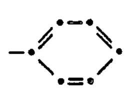 | $C\equiv C-CH_3$ | $CH_3$ | |
| 1.122 | 2-Cl | H | 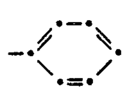 | 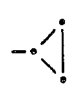 | $CH_2F$ | |
| 1.123 | 2-Cl | H | 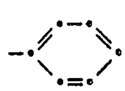 | $CH_2F$ | $CH_3$ | |

Fortsetzung: Tabelle 1

| Verb. Nr. | X | Y | $R_2$ | $R_4$ | $R_5$ | physikalische Konstante |
|---|---|---|---|---|---|---|
| 1.124 | 2-Cl | H | 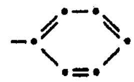 | $C{\equiv}C-CH_3$ | $CH_3$ | |
| 1.125 | $2-CH_2CH_3$ | H | $CH_3$ | 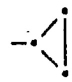 | $CH_3$ | $n_D^{49}$: 1,5959 |
| 1.126 | $2-CH_2CH_3$ | $4-CH_2CH_3$ | $CH_3$ | $CH_2OCH_3$ | $CH_3$ | |
| 1.127 | $2-CH_2CH_3$ | H | $CH_3$ | $CH_2F$ | 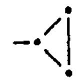 | |
| 1.128 | $2-CH_2CH_3$ | H | $CH_3$ | $C{\equiv}C-CH_3$ | $CH_3$ | |
| 1.129 | $2-CH_2CH_3$ | H | $CH_3$ | $CH_2OCH_3$ | $CH_3$ | |
| 1.130 | $3-CH_3$ | $4-CH_3$ | $CH_3$ | $CH_2F$ | 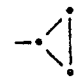 | |
| 1.131 | $2-CH(CH_3)_2$ | H | $CH_3$ | 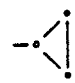 | $CH_3$ | |
| 1.132 | $2-CH_3$ | $4-C(CH_3)_4$ | $CH_3$ | 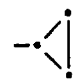 | $CH_3$ | |
| 1.133 | $2-CH_3$ | $4-SCH_3$ | $CH_3$ | 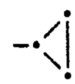 | $CH_3$ | |
| 1.134 | $2-CH(CH_3)_2$ | H | $CH_3$ | $CH_2OCH_3$ | $CH_3$ | |
| 1.135 | $2-CH_3$ | $4-SOCH_3$ | $CH_3$ | 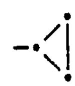 | $CH_3$ | |
| 1.136 | $2-CH(CH_3)_2$ | H | $CH_3$ | $CH_2F$ | 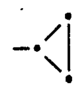 | |
| 1.137 | $2-CH_3$ | $4-SO_2CH_3$ | $CH_3$ | 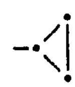 | $CH_3$ | |
| 1.138 | 2-Cl | $5-CH_3$ | $CH_3$ | 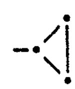 | $CH_3$ | |
| 1.139 | $2-CH_3$ | H | $CH_3$ | 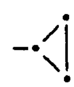 | H | |
| 1.140 | 2-Cl | H | $CH_3$ | $CH_2OCH_3$ | $SCH_3$ | |
| 1.141 | 2-Cl | $5-CH_3$ | $CH_3$ | $CH_2F$ | 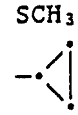 | |
| 1.142 | $2-CH_3$ | 4-Cl | $CH_3$ | $C{\equiv}C-CH_3$ | $CH_3$ | |
| 1.143 | 2-Cl | 4-Cl | $CO_2C_2H_5$ | 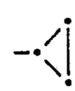 | $CH_3$ | |
| 1.144 | $2-CH_3$ | 4-Cl | $CH_3$ | $CH_2OCH_3$ | $CH_3$ | |

<u>Fortsetzung:</u> Tabelle 1

| Verb. Nr. | X | Y | $R_2$ | $R_4$ | $R_5$ | physikalische Konstante |
|---|---|---|---|---|---|---|
| 1.145 | 2-$CH_3$ | 4-Cl | $CH_3$ | $CH_2F$ | 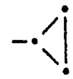 | |
| 1.146 | 2-$CH_3$ | 4-Cl | $CH_3$ | $CH_2F$ | $CH_3$ | |
| 1.147 | 2-Cl | 6-Cl | $CH_3$ | 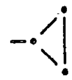 | $CH_3$ | |
| 1.148 | 2-OH | H | $CH_3$ | 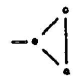 | $CH_3$ | Smp. 132-136°C |
| 1.149 | H | H | 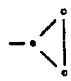 | 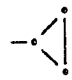 | $CH_3$ | $n_D^{24,5}$: 1,6262 |
| 1.150 | 2-$CH_3$ | 4-OH | $CH_3$ | 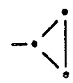 | $CH_3$ | Smp. 160-163°C |
| 1.151 | 2-$CH_3$ | H | $CH_3$ | 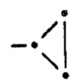 | Allyl | |
| 1.152 | 2-$CH_3$ | H | $CH_3$ | $CH_2OCH_3$ | Allyl | |
| 1.153 | 2-$CH_3$ | H | $CH_3$ | $CH_2F$ | Allyl | |
| 1.154 | 2-$CH_3$ | H | $CH_3$ | $CH_2OCH_3$ | OH | Smp. 80-82°C |
| 1.155 | 2-F | H | $CH_3$ | $CF_3$ | $CH_3$ | Smp. 147-148°C |
| 1.156 | 2-$CH_3$ | 5-$CH_3$ | $CH_3$ | $CF_3$ | $CH_3$ | Smp. 99-100°C |
| 1.157 | 2-$CH_3$ | 5-$CH_3$ | $CH_3$ | $CH_2OCH_3$ | $OCH_3$ | $n_D^{36}$: 1,5809 |
| 1.158 | 2-$NO_2$ | H | $CH_3$ | $CF_3$ | $CH_3$ | Smp. 155-160°C |
| 1.159 | 2-$CF_3$ | H | $CH_3$ | $CF_3$ | $CH_3$ | Smp. 129-130°C |
| 1.160 | 2-Cl | H | $CH_3$ | $CH_2OCH_3$ | OH | Smp. 88-92°C |
| 1.161 | 2-Cl | H | $CH_3$ | $CH_2OCH_3$ | Cl | Smp. 122-123°C |
| 1.162 | 2-$OCHF_2$ | H | $CH_3$ | $CH_2OCH_3$ | OH | Smp. 94-95°C |
| 1.163 | 2-$OCHF_2$ | H | $CH_3$ | $CH_2OCH_3$ | Cl | Smp. 85-88°C |
| 1.164 | 2-$OCHF_2$ | H | $CH_3$ | $CF_3$ | $CH_3$ | Smp. 115-116°C |
| 1.165 | 2-Cl | H | $CH_3$ | $CH_2OCH_3$ | $OCH_3$ | Smp. 102-103°C |
| 1.166 | 2-$CH_3$ | 5-$CH_3$ | $CH_3$ | $CH_2OCH_3$ | $SCH_3$ | Smp. 127-128°C |
| 1.167 | 2-$CH_3$ | 4-F | $CH_3$ | 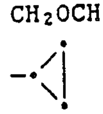 | $CH_3$ | Smp. 161-162°C |
| 1.168 | 2-$CH_3$ | H | $CH_3$ | $SCH_3$ | $CH_3$ | |
| 1.169 | 2-Br | H | $CH_3$ | $SCH_3$ | $CH_3$ | |
| 1.170 | 2-F | H | $CH_3$ | $SCH_3$ | $CH_3$ | |
| 1.171 | 2-$CH_3$ | H | $CH_3$ | S-$C_2H_5$ | $CH_3$ | |
| 1.172 | 2-$CH_3$ | 5-$CH_3$ | $CH_3$ | S-$CH_3$ | $CH_3$ | |
| 1.173 | 2-$CH_3$ | H | $CH_3$ | 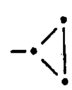 | $SCH_3$ | |

Tabelle 2: Verbindungen der Formel

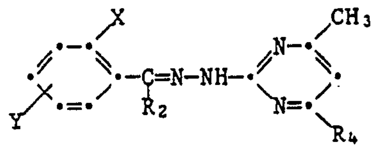

| Verb. Nr. | X | Y | $R_2$ | $R_4$ | Isomere Form | physikalische Konstante |
|---|---|---|---|---|---|---|
| 2.1 | $CH_3$ | H | $CH_3$ | 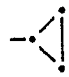 | Z | Smp. 96-98°C |
| 2.2 | $CH_3$ | H | $CH_3$ | 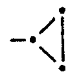 | E | Smp. 121-122°C |
| 2.3 | $CH_3$ | H | $CH_3$ | $CH_2F$ | E | |
| 2.4 | $CH_3$ | H | $CH_3$ | $CH_2F$ | Z | |
| 2.5 | $CH_3$ | H | $CH_3$ | $CF_3$ | Z | Smp. 136-137°C |
| 2.6 | $CH_3$ | H | $CH_3$ | $CF_3$ | E | Smp. 150-151°C |
| 2.7 | $CH_3$ | H | $CH_3$ | $CH_2OCH_3$ | E | $n_D^{27}$: 1,6015 |
| 2.8 | $CH_3$ | H | $CH_3$ | $CH_2OCH_3$ | Z | $n_D^{27}$: 1,5712 |
| 2.9 | $OCHF_2$ | H | $CH_3$ | 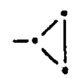 | Z | Smp. 147-150°C |
| 2.10 | $OCHF_2$ | H | $CH_3$ | 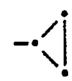 | E | Smp. 93-96°C |
| 2.11 | $CH_3$ | H | $CH_3$ | $C{\equiv}C{-}CH_3$ | E | |
| 2.12 | $CH_3$ | H | $CH_3$ | $C{\equiv}C{-}CH_3$ | Z | |
| 2.13 | $CH_3$ | H | $CH_3$ | 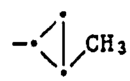 | E | |
| 2.14 | $CH_3$ | H | $CH_3$ | 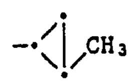 | Z | |
| 2.15 | Cl | H | $CH_3$ | $CF_3$ | E | Smp. 175-177°C |
| 2.16 | Cl | H | $CH_3$ | $CF_3$ | Z | $n_D^{25}$: 1,5618 |
| 2.17 | $NO_2$ | H | $CH_3$ | 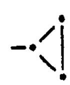 | E | Smp. 144-146°C |
| 2.18 | $NO_2$ | H | $CH_3$ | 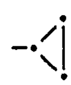 | Z | Smp. 144-146°C |
| 2.19 | H | H | 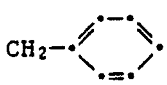 | 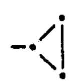 | E | Smp. 91-92°C |
| 2.20 | $CH_2CH_3$ | H | $CH_3$ | 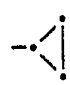 | E | Smp. 83-85°C |
| 2.21 | $CH_3$ | 4-$OCHF_2$ | $CH_3$ | 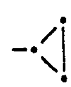 | E | Smp. 115-116°C |

Tabelle 2 (Fortsetzung)

| Verb. Nr. | X | Y | $R_2$ | $R_4$ | Isomere Form | physikalische Konstante |
|---|---|---|---|---|---|---|
| 2.22 | Br | H | $CH_3$ | $CF_3$ | E | Smp. 172-173°C |
| 2.23 | $NO_2$ | H | $CH_3$ | $CF_3$ | E | Smp. 191-192°C |
| 2.24 | $2-CH_3$ | $4-CH_3$ | $CH_3$ | $CF_3$ | E | Smp. 141-142°C |
| 2.25 | $2-CH_3$ | $4-CH_3$ | $CH_3$ | $CF_3$ | Z | Smp. 104-105°C |
| 2.26 | $2-CH_3$ | $4-CH_3$ | $CH_3$ | 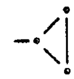 | Z | Smp. 123-125°C |

Tabelle 3: Verbindungen der Formel

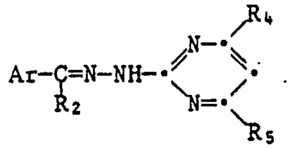

| Verb. Nr. | Ar | $R_2$ | $R_4$ | $R_5$ | physikalische Konstante |
|---|---|---|---|---|---|
| 3.1 | 2,4,5-$(CH_3)_3$-Phenyl | $CH_3$ | 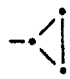 | $CH_3$ | |
| 3.2 | 2,3,4-$Cl_3$-Phenyl | $CH_3$ | $CH_2OCH_3$ | $CH_3$ | |
| 3.3 | 2,3,4,5-$(CH_3)_4$-Phenyl | $CH_3$ | 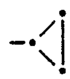 | $CH_3$ | |
| 3.4 | α-Naphthyl | $CH_3$ | $C\equiv C-CH_3$ | $CH_3$ | |
| 3.5 | 2,5-$(CH_3)_2$-4-Cl-Phenyl | $CH_3$ | 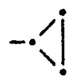 | $CH_3$ | |
| 3.6 | 2,4-$(CH_3)_2$-α-Naphthyl | $CH_3$ | $CH_2F$ | 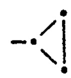 | |
| 3.7 | β-Naphthyl | $CH_3$ | 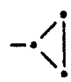 | $CH_3$ | Smp. 133-138°C |
| 3.8 | 2-$CH_3$-4,5-$Cl_2$-Phenyl | $CH_3$ | $CH_2F$ | 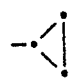 | |
| 3.9 | 2,4-$Cl_2$-5-$CH_3$-Phenyl | $CH_3$ | 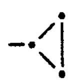 | $CH_3$ | |
| 3.10 | 1,4-$(CH_3)_2$-β-Naphthyl | $CH_3$ | $CH_2OCH_3$ | $CH_3$ | |
| 3.11 | 2,3,4,5-$(CH_3)_4$-Phenyl | $CH_3$ | $CH_2F$ | $CH_3$ | |
| 3.12 | α-Naphthyl | $CH_3$ | 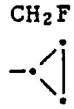 | $CH_3$ | Smp. 123-125°C |
| 3.13 | 2,4-$(CH_3)_2$-α-Naphthyl | $CH_3$ | 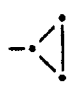 | $CH_3$ | |
| 3.14 | 2,4,5-$(CH_3)_3$-Phenyl | $CH_3$ | $C\equiv C-CH_3$ | $CH_3$ | |
| 3.15 | α-Naphthyl | $CH_3$ | $CF_3$ | $CH_3$ | |
| 3.16 | 2,4,5-$(CH_3)_3$-Phenyl | $CH_3$ | $CH_2OCH_3$ | $CH_3$ | |
| 3.17 | β-Naphthyl | $CH_3$ | $CH_2F$ | 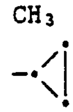 | |
| 3.18 | 2-$CH_3$-4,5-$Cl_2$-Phenyl | $CH_3$ | 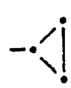 | $CH_3$ | |
| 3.19 | 2,4,5-$(CH_3)_3$-Phenyl | $CH_3$ | $CH_2F$ | 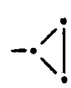 | |

Fortsetzung: Tabelle 3

| Verb. Nr. | Ar | $R_2$ | $R_4$ | $R_5$ | physikalische Konstante |
|---|---|---|---|---|---|
| 3.20 | β-Naphthyl | $CH_3$ | $CH_2F$ | $CH_3$ | |
| 3.21 | 2,4-$Cl_2$-5-$CH_3$-Phenyl | $CH_3$ | $CH_2OCH_3$ | $CH_3$ | |
| 3.22 | 2,3,4,5-$(CH_3)_4$-Phenyl | $CH_3$ | $C{\equiv}C-CH_3$ | $CH_3$ | |
| 3.23 | α-Naphthyl | $CH_3$ | $CH_2OCH_3$ | $CH_3$ | |
| 3.24 | 2,3,4-$Cl_3$-Phenyl | $CH_3$ | 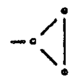 | $CH_3$ | |
| 3.25 | 2-$CH_3$-α-Naphthyl | $CH_3$ | 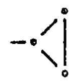 | $CH_3$ | |
| 3.26 | 2,4-$(CH_3)_2$-α-Naphthyl | $CH_3$ | 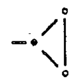 | $CH_3$ | |
| 3.27 | 2-$CH_3$-α-Naphthyl | $CH_3$ | $CH_2OCH_3$ | $CH_3$ | |
| 3.28 | 2,4-$(CH_3)_2$-α-Naphthyl | $CH_3$ | $CH_2OCH_3$ | $CH_3$ | |
| 3.29 | 2,3-$(CH_3)_2$-α-Naphthyl | $CH_3$ | 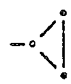 | $CH_3$ | |
| 3.30 | 2,3-$(CH_3)_2$-α-Naphthyl | $CH_3$ | $CH_2OCH_3$ | $CH_3$ | |
| 3.31 | 2,4,6-$(CH_3)_3$-Phenyl | $CH_3$ | 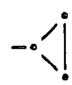 | $CH_3$ | Smp. 139-140°C |

Tabelle 4: Verbindungen der Formel

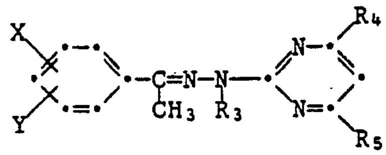

| Verb. Nr. | X | Y | $R_3$ | $R_4$ | $R_5$ | physikalische Konstante |
|---|---|---|---|---|---|---|
| 4.1 | 2-$CH_3$ | H | $CH_3$ | 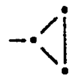 | $CH_3$ | |
| 4.2 | 2-$CH_3$ | H | $COCH_3$ | $CH_2OCH_3$ | $CH_3$ | |
| 4.3 | 2-Cl | H | $COCHCl_2$ | 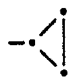 | $CH_3$ | |
| 4.4 | 2-$CH_3$ | H | $CH_3$ | 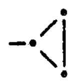 | $CH_2F$ | |
| 4.5 | 2-$CH_3$ | 4-$CH_3$ | $CH_2CH_3$ | 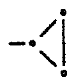 | $CH_3$ | |
| 4.6 | 2-$CH_3$ | H | $CH_3$ | $C{\equiv}C-CH_3$ | $CH_3$ | |
| 4.7 | 2-Cl | H | $COCH_2OCH_3$ | 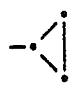 | $CH_3$ | |
| 4.8 | 2-$CH_3$ | H | $COCH_2OCH_3$ | 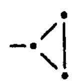 | $CH_3$ | |
| 4.9 | 2-F | H | $CH_3$ | 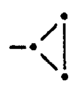 | $CH_3$ | |
| 4.10 | 2-$CH_3$ | H | n-$C_3H_7$ | $CH_2OCH_3$ | $CH_3$ | |
| 4.11 | 2-$CH_3$ | H | $COCCl_2CH_3$ | 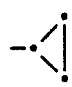 | $CH_3$ | |
| 4.12 | 2-$CH_3$ | 4-$CH_3$ | $CH_3$ | 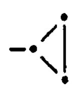 | $CH_3$ | |
| 4.13 | 2-$CH_3$ | 4-$OCH_3$ | $CH_3$ | $CH_2OCH_3$ | $CH_3$ | |
| 4.14 | 2-$CH_3$ | 4-Cl | $CH_3$ | $C{\equiv}C-CH_3$ | $CH_3$ | |
| 4.15 | 2-$CH_3$ | H | $CH_2CH_3$ | $CH_2OCH_3$ | $OCH_3$ | |

Tabelle 5: Verbindungen der Formel

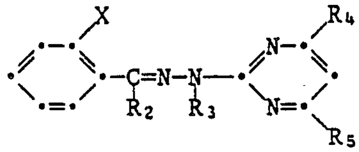

| Verb. Nr. | X | $R_2$ | $R_3$ | $R_4$ | $R_5$ | physikalische Konstante |
|---|---|---|---|---|---|---|
| 5.1 | $CH_3$ | 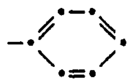 | $CH_3$ | 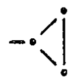 | $CH_3$ | |
| 5.2 | H | 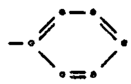 | $CH_2CH_3$ | 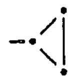 | $CH_3$ | $n_D^{25}$: 1,6145 |
| 5.3 | H | $CH_2$- 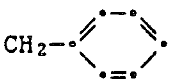 | $COCHCl_2$ | 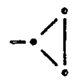 | $CH_3$ | |
| 5.4 | Cl | 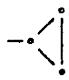 | $CH_2CH_3$ | $CH_2OCH_3$ | $CH_3$ | |
| 5.5 | $CH_3$ | 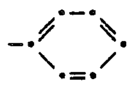 | $CH_3$ | $C{\equiv}C-CH_3$ | $CH_3$ | |
| 5.6 | $CH_3$ | $CF_3$ | $COCH_2OCH_3$ | 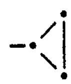 | $CH_3$ | |
| 5.7 | $CH_3$ | $CH_2CH_3$ | $CH_3$ | 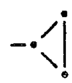 | $CH_3$ | |
| 5.8 | $CH_3$ | $CH_2CH_3$ | $COCHCl_2$ | 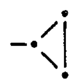 | $CH_3$ | |
| 5.9 | Cl | $CH_2CF_3$ | $CH_2CH_3$ | 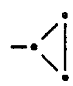 | $CH_3$ | |
| 5.10 | $CH_3$ | 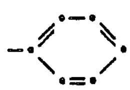 | $COCHCl_2$ | 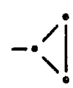 | $CH_3$ | |

Sowie ferner die folgenden Verbindungen:

| | | physikalische Konstante |
|---|---|---|
| 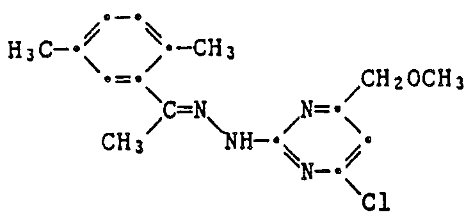 | E-Form: | Smp. 144-145°C |

| | | physikalische Konstante |
|---|---|---|
| 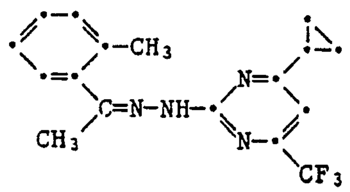 | E-Form:<br>Z-Form: | Smp. 104°C<br>$n_D^{50}$: 1,5484 |
| 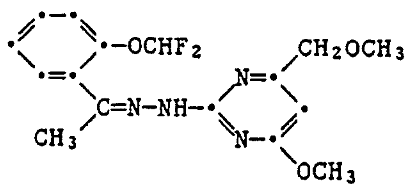 | E-Form:<br>Z-Form: | Smp. 123-124°C<br>Smp. 111-113°C |

2. Formulierungsbeispiele für flüssige Wirkstoffe der Formel I % = Gewichtsprozent)

| 2.1. Emulsionskonzentrate | a) | b) | c) |
|---|---|---|---|
| Wirkstoff aus der Tabelle 1 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff aus der Tabelle 1 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190° C) | - | - | 94 % | - |
| (MG = Molekulargewicht) | | | | |

Die Lösungen sind zur Anwendung in Form kleinster Tropfen geeignet.

| 2.3. Granulate | a) | b) |
|---|---|---|
| Wirkstoff aus Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4. Stäubemittel | a) | b) |
|---|---|---|
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| 2.5. Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6. Emulsions-Konzentrat | |
|---|---|
| Wirkstoff aus der Tabelle 1 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7. Stäubemittel | a) | b) |
|---|---|---|
| Wirkstoff aus der Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.8. Extruder Granulat | |
|---|---|
| Wirkstoff aus der Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9. Umhüllungs-Granulat | |
|---|---|
| Wirkstoff aus der Tabelle 1 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10. Suspensions-Konzentrat | |
|---|---|
| Wirkstoff aus der Tabelle 1 | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## 3. Biologische Beispiele

### Beispiel 3.1: Wirkung gegen Phytophthora auf Tomatenpflanzen

a) Residual-protektive Wirkung

Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20 °C.

b) Residual-kurative Wirkung

Tomatenpflanzen werden nach 3-wöchiger Anzucht mit einer Sporangiensuspension des Pilzes infiziert.

Nach einer Inkubation von 22 Stunden in einer Feuchtkammer bei 90-100 % relativer Luftfeuchtigkeit und 20 °C werden die infizierten Pflanzen getrocknet und mit einer aus Spritzpulver des Wirkstoffes hergestellten Spritzbrühe (0,02 % Aktivsubstanz) besprüht. Nach dem Antrocknen des Spritzbelages werden die behandelten Pflanzen wieder in die Feuchtkammer begracht. Die Beurteilung des Pilzbefalls erfolgt 5 Tage nach der Infektion.

c) Systemische Wirkung

Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kam. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20 °C.

Verbindungen aus den Tabellen zeigen gegen Phytophthora-Befall gute Wirksamkeit (Befall 20 % oder weniger). So reduzieren z.B. die Verbindungen Nr. 1.1, 1.5, 1.9, 1.13, 2.1, 2.2, 2.16 und 2.19 den Phytophthora-Befall auf 0 bis 5 %. Unbehandelte aber infizierte Kontrollpflanzen weisen dagegen einen Phytophthora-Befall von 100 % auf.

Beispiel 3.2: Wirkung gegen Plasmopara viticola auf Reben Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20 °C wird der Pilzbefall beurteilt.

Verbindungen aus den Tabellen zeigen gegen Plasmopara gute Wirksamkeit (Pilzbefall unter 20 %). So reduzieren z.B. die Verbindungen Nr. 1.35 und 1.39 den Plasmopara-Befall auf 0 bis 5 %. Unbehandelte aber infizierte Kontrollpflanzen weisen dagegen einen Plasmopara-Befall von 100 % auf.

Beispiel 3.3: Wirkung gegen Cercospora arachidicola auf Erdnusspflanzen Residual protektive Wirkung

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Verbindungen aus den Tabellen zeigen gegen Cercospora gute Wirkamkeit (Pilzbefall unter 20 %). So reduzieren z.B. die Verbindungen Nr. 1.1, 1.2, 1.9, 1.11, 1.13, 1.35, 1.45, 1.46, 1.50, 1.116, 2.1, 2.2, 2.9, 2.17 und 2.18 den Cercospora-Befall auf 0 bis 5 %. Unbehandelte aber infizierte Kontrollpflanzen weisen dagegen einen Cercospora-Befall von 100 % auf.

Beispiel 3.4: Wirkung gegen Botrytis cinerea an Apfelfrüchten Residual-protektive Wirkung

Künstlich verletzte Aepfel werden behandelt, indem eine aus Spritzpulver der Wirksubstanz hergestellte Spritzbrühe (0,02 % Aktivsubstanz) auf die Verletzungsstellen aufgetropft wird. Die behandelten Früchte werden anschliessend mit einer Sporensuspension des Pilzes inokuliert und während einer Woche bei hoher Luftfeuchtigkeit und ca. 20 °C inkubiert. Bei der Auswertung werden die angefaulten Verletzungsstellen gezählt und daraus die fungizide Wirkung der Testsubstanz abgeleitet.

Verbindungen aus den Tabellen sind gegen Botrytis gut wirksam. Unbehandelte aber infizierte Kontrollpflanzen weisen dagegen einen Pilzbefall von 100% auf.

Verbindungen aus den Tabellen zeigen gegen Botrytis gute Wirkamkeit. So reduzieren z.B. die Verbindungen Nr. 1.2, 1.11, 1.13, 1.35, 1.45, 1.125, 1.157, 1.116, 2.1 und 3.7 den Botrytis-Befall auf 0 bis 5 %. Unbehandelte aber infizierte Kontrollpflanzen weisen dagegen einen Botrytis-Befall von 100 % auf.

Beispiel 3.5: Wirkung gegen Rhizoctonia solani (Bodenpilz auf Reispflanzen)

a) Protektiv-lokale Bodenapplikation

12 Tage alte Reispflanzen werden mit einer aus einer Formulierung des Wirkstoffes hergestellten Spritzbrühe (0,006 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27° C (Tag), bzw. 23° C (Nacht) und 100 % rel. Luftfeuchtigkeit (Feuchtkasten) in der Klimakammer wird der Pilzbefall auf Blattscheide, Blättern und Stengel beurteilt.

b) Protektiv-lokale Blattapplikation

12 Tage alte Reispflanzen werden mit einer aus einer Formulierung der Wirkstoffe hergestellten Spritzbrühe (0,02 % Aktivsubstanz) besprüht. Einen Tag später werden die behandelten Pflanzen mit einer Suspension von Myzel und Sklerotien von R. solani infiziert. Nach 6-tägiger Inkubation bei 27° C (Tag), bzw. 23° C (Nacht) und 100 % rel. Luftfeuchtigkeit (Feuchtkasten) in der Klimakammer wird der Pilzbefall auf Blattscheide, Blättern und Stengel beurteilt.

Verbindungen aus den Tabellen sind gegen Rhizoctonia gut wirksam. So reduzieren z.B. die Verbindungen Nr. 1.1, 1.9, 1.31, 1.35, 1.45, 1.50, 1.166, 2.1, 2.20, 2.21 und 3.12 den Rhizoctonia-Befall auf 0 bis 5 %. Unbehandelte aber infizierte Kontrollpflanzen weisen dagegen einen Befall von 100 % auf.

Beispiel 3.6: Wirkung gegen Pyricularia oryzae auf Reispflanzen

a) Residual-protektive Wirkung

Reispflanzen werden nach zweiwöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24° C wird der Pilzbefall beurteilt.

b) Systemische Wirkung

Zu zwei Wochen alten in Blumentöpfen wachsenden Reispflanzen wird eine aus Spritzpulver des Wirkstoffes hergestellte Spritzbrühe gegossen (0,006 % Aktivsubstanz, bezogen auf das Erdvolumen). Darauf werden die Töpfe mit Wasser soweit gefüllt, dass die untersten Stengelteile der Reispflanzen im Wasser stehen. Nach 48 Stunden werden die behandelten Reispflanzen mit einer Konidiensuspension des Pilzes infiziert. Nach einer Inkubation der infizierten Pflanzen während 5 tagen bei 95-100 % relativer Luftfeuchtigkeit und ca. 24° wird der Pilzbefall beurteilt.

c) Kurative Wirkung

Reispflanzen werden nach zweiwöchiger Anzucht mit einer Konidiensuspension des Pilzes infiziert. Nach 1 bis 2 Tagen Inkubation bei 95-100 % relativer Luftfeuchtigkeit und 24° C werden die Pflanzen mit einer aus Spritzpulver des Wirkstoffes hergestellten Spritzbrühe (0,02 % Aktivsubstanz) besprüht. Nach weiteren 5 Tagen Inkubation bei 95-100 % relativer Luftfeuchtigkeit und 24° C wird der Pilzbefall beurteilt.

Verbindungen aus den Tabellen zeigen nachhaltige Wirkung gegen den Pyricularia-Pilz (unter 20 % Befall). So reduzieren z.B. die Verbindungen Nr. 1.1, 1.9, 1.46, 2.1, 2.10 und 2.18 den Pyricularia-Befall auf 0 bis 5 %. Unbehandelte aber infizierte Kontrollpflanzen weisen dagegen einen Befall von 100 % auf.

Beispiel 3.7: Wirkung gegen Helminthosporium gramineum

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen. Die Verbindungen aus den Tabellen verhindern den Pilzbefall weitgehend (unter 20 % Befall). So reduzieren z.B. die Verbindungen Nr. 1.1, 1.9, 1.45, 1.46, 1.66 und 2.17 den Helminthosporium-Befall auf 0 bis 5 %. Unbehandeltes aber infiziertes Kontroll-Saatgut weist dagegen einen Befall von 100 % auf.

Beispiel 3.8: Wirkung gegen Fusarium nivale

Weizenkörner werden mit einer Sporensuspension des Pilzes kontaminiert und wieder angetrocknet. Die kontaminierten Körner werden mit einer aus Spritzpulver hergestellten Suspension der Testsubstanz gebeizt (600 ppm Wirkstoff bezogen auf das Gewicht der Samen). Nach zwei Tagen werden die Körner auf geeignete Agarschalen ausgelegt und nach weiteren vier Tagen wird die Entwicklung der Pilzkolonien um die Körner herum beurteilt. Anzahl und Grösse der Pilzkolonien werden zur Beurteilung der Testsubstanz herangezogen.

Bei den Körnern, die mit einem Spritzpulver behandelt worden sind, das als Wirkstoff eine Verbindung aus den Tabellen enthält, wird die Entwicklung der Pilzkolonien weitgehend unterdrückt (unter 20 % Befall). So reduzieren z.B. die Verbindungen Nr. 1.1, 1.9 und 1.45 den Fusarium-Befall auf 0 bis 10 %. Unbehandeltes aber infiziertes Kontroll-Saatgut weist dagegen einen Befall von 100 % auf.

## Ansprüche

1. Verbindungen der Formel I

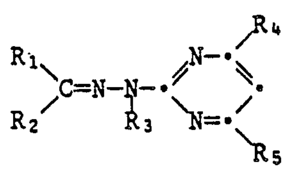

(I)

in welcher bedeuten:

$R_1$ Phenyl, Naphthyl, 1- bis 5-fach mit $R_6$ substituiertes Phenyl oder 1-bis 5-fach mit $R_6$ substituiertes Naphthyl;

$R_2$ $C_1$-$C_6$-Alkyl, mindestens einfach mit Halogen, $C_1$-$C_3$-Alkoxy und/oder $C_3$-$C_6$-Cycloalkyl substituiertes $C_1$-$C_6$-Alkyl, $C_3$-$C_6$-Cycloalkyl, $C_1$-$C_4$-Alkoxycarbonyl, Phenyl, 1- bis 3-fach mit Halogen, $C_1$-$C_3$-Alkoxy und/oder $C_1$-$C_3$-Alkyl substituiertes Phenyl, Benzyl oder 1- bis 3-fach mit Halogen, $C_1$-$C_3$-Alkoxy und/oder $C_1$-$C_3$-Alkyl substituiertes Benzyl;

$R_3$ Wasserstoff, $C_1$-$C_6$-Alkyl oder den Rest CO-$R_7$;

$R_4$ $C_3$-$C_6$-Cycloalkyl, 1- bis 3-fach mit Halogen und/oder Methyl substituiertes $C_3$-$C_6$-Cycloalkyl, mindestens einfach mit Halogen, Hydroxy und/oder Cyano substituiertes $C_1$-$C_4$-Alkyl, $C_2$-$C_5$-Alkinyl, $C_1$-$C_4$-Alkylthio oder den Rest $CH_2XR_8$;

$R_5$ Wasserstoff, $C_1$-$C_6$-Alkyl, mindestens einfach mit Halogen, Hydroxy, Cyano und/oder $C_1$-$C_3$-Alkoxy substituiertes $C_1$-$C_6$-Alkyl, $R_5$ ferner $C_3$-$C_5$-Alkenyl, Halogen, Hydroxy, $C_1$-$C_3$-Alkoxy, $C_1$-$C_3$-Haloalkoxy, $C_3$-$C_6$-Cycloalkyl oder $C_1$-$C_3$-Alkylthio;

$R_6$ unabhängig voneinander $C_1$-$C_6$-Alkyl, mit $C_1$-$C_3$-Alkoxy substituiertes $C_1$-$C_3$-Alkyl, $C_1$-$C_4$-Alkoxy, $C_1$-$C_3$-Haloalkyl, $C_1$-$C_3$-Haloalkoxy, Halogen, Cyano, Nitro, Hydroxy oder den Rest $(S(O)_n$-$C_1$-$C_4$-Alkyl;

$R_7$ $C_1$-$C_6$-Alkyl oder ein mindestens einfach mit Halogen und/oder $C_1$-$C_3$-Alkoxy substituiertes $C_1$-$C_6$-Alkyl;

$R_8$ $C_1$-$C_6$-Alkyl, mindestens einfach mit Halogen, Hydroxy, Cyano und/oder $C_1$-$C_4$-Alkoxy substituiertes $C_1$-$C_6$-Alkyl, $C_3$-$C_6$-Alkenyl, $C_3$-$C_6$-Alkinyl oder $C_3$-$C_6$-Cycloalkyl;

X Sauerstoff oder Schwefel und

n 0, 1 oder 2;

unter Einschluss ihrer Säureadditionsverbindungen und ihrer Metallsalzkomplexe.

2. Verbindungen der Formel I gemäss Anspruch 1, worin bedeuten:

$R_1$ Phenyl, Naphthyl, bis zu 5-fach mit $R_6$ substituiertes Phenyl oder bis zu 5-fach mit $R_6$ substituiertes Naphthyl;

$R_2$ $C_1$-$C_6$-Alkyl, 1- bis 5-fach mit Halogen oder 1- bis 2-fach mit $C_1$-$C_3$-Alkoxy und/oder 1-fach mit $C_3$-$C_6$-Cycloalkyl substituiertes $C_1$-$C_6$-Alkyl, $C_3$-$C_6$-Cycloalkyl, Phenyl, 1- bis 3-fach mit Halogen, $C_1$-$C_3$-Alkoxy und/oder $C_1$-$C_3$-Alkyl substituiertes Phenyl, Benzyl oder 1-bis 3-fach mit Halogen, $C_1$-$C_3$-Alkoxy und/oder $C_1$-$C_3$-Alkyl substituiertes Benzyl;

$R_3$ Wasserstoff, $C_1$-$C_3$-Alkyl oder den Rest CO-$R_7$;

$R_4$ $C_3$-$C_6$-Cycloalkyl, 1- bis 3-fach mit Halogen und/oder Methyl substituiertes $C_3$-$C_6$-Cycloalkyl, mindestens einfach mit Halogen, Hydroxy und/oder Cyano substituiertes $C_1$-$C_4$-Alkyl, $C_2$-$C_5$-Alkinyl, $C_1$-$C_4$-Alkylthio oder den Rest $CH_2XR_8$;

$R_5$ Wasserstoff, $C_1$-$C_6$-Alkyl, mindestens einfach mit Halogen, Hydroxy, Cyano und/oder $C_1$-$C_3$-Alkoxy substituiertes $C_1$-$C_6$-Alkyl, $R_5$ ferner $C_3$-$C_5$-Alkenyl, Halogen, Hydroxy, $C_1$-$C_3$-Alkoxy, $C_1$-$C_3$-Haloalkoxy, $C_3$-$C_6$-Cycloalkyl oder $C_1$-$C_3$-Alkylthio;

$R_6$ unabhängig voneinander $C_1$-$C_4$-Alkyl, $C_1$-$C_3$-Alkoxy, $C_1$-$C_3$-Haloalkyl, $C_1$-$C_3$-Haloalkoxy, Halogen, Nitro oder den Rest $S(O)_n$-$C_1$-$C_3$-Alkyl;

$R_7$ $C_1$-$C_6$-Alkyl oder mindestens einfach mit Halogen und/oder $C_1$-$C_3$-Alkoxy substituiertes $C_1$-$C_6$-Alkyl;

$R_8$ $C_1$-$C_6$-Alkyl, mindestens einfach mit Halogen, Hydroxy und/oder $C_1$-$C_4$-Alkoxy substituiertes $C_1$-$C_6$-Alkyl, $C_3$-$C_6$-Alkenyl, $C_2$-$C_6$-Alkinyl oder $C_3$-$C_6$-Cycloalkyl;

X Sauerstoff oder Schwefel und

n 0, 1 oder 2.

3. Verbindungen der Formel I gemäss Anspruch 1, worin bedeuten:

$R_1$ Phenyl, Naphthyl, bis zu 5-fach mit $R_6$ substituiertes Phenyl oder bis zu 3-fach mit $R_6$ substituiertes Naphthyl;

$R_2$ $C_1$-$C_6$-Alkyl, 1- bis 5-fach mit Halogen oder 1- bis 2-fach mit $C_1$-$C_3$-Alkoxy und/oder 1-fach mit $C_3$-$C_6$-Cycloalkyl substituiertes $C_1$-$C_6$-Alkyl, $C_3$-$C_6$-Cycloalkyl, Phenyl, 1- bis 3-fach mit Halogen, $C_1$-$C_3$-Alkoxy und/oder $C_1$-$C_3$-Alkyl substituiertes Phenyl, Benzyl oder 1-bis 3-fach mit Halogen, $C_1$-$C_3$-Alkoxy und/oder $C_1$-$C_3$-Alkyl substituiertes Benzyl;

$R_3$ Wasserstoff, $C_1$-$C_3$-Alkyl oder den Rest CO-$R_7$;

$R_4$ $C_3$-$C_6$-Cycloalkyl, 1- bis 3-fach mit Halogen und/oder Methyl substituiertes $C_3$-$C_6$-Cycloalkyl, $C_2$-$C_5$-Alkinyl, $C_1$-$C_4$-Alkylthio oder den Rest $CH_2XR_8$;

$R_5$ Wasserstoff, $C_1$-$C_6$-Alkyl, mindestens einfach mit Halogen, Hydroxy, Cyano und/oder $C_1$-$C_3$-Alkoxy substituiertes $C_1$-$C_6$-Alkyl, $R_5$ ferner $C_3$-$C_5$-Alkenyl, Halogen, Hydroxy, $C_1$-$C_3$-Alkoxy, $C_1$-$C_3$-Haloalkoxy, $C_3$-$C_6$-Cycloalkyl oder $C_1$-$C_3$-Alkylthio;

$R_6$ unabhängig voneinander $C_1$-$C_4$-Alkyl, $C_1$-$C_3$-Alkoxy, $C_1$-$C_3$-Haloalkyl, $C_1$-$C_3$-Haloalkoxy, Halogen, Nitro oder den Rest $S(O)_n$-$C_1$-$C_3$-Alkyl;

$R_7$ $C_1$-$C_6$-Alkyl oder mindestens einfach mit Halogen und/oder $C_1$-$C_3$-Alkoxy substituiertes $C_1$-$C_6$-Alkyl;

$R_8$ $C_1$-$C_6$-Alkyl, mindestens einfach mit Halogen, Hydroxy und/oder $C_1$-$C_4$-Alkoxy substituiertes $C_1$-$C_6$-Alkyl, $C_3$-$C_6$-Alkenyl, $C_3$-$C_6$-Alkinyl oder $C_3$-$C_6$-Cycloalkyl;

X Sauerstoff oder Schwefel und

n 0, 1 oder 2.

4. Verbindungen der Formel I gemäss Anspruch 1, worin bedeuten:

$R_1$ Phenyl, Naphthyl, bis zu 3-fach mit $R_6$ substituiertes Phenyl oder bis zu 3-fach mit $R_6$ substituiertes Naphthyl;

$R_2$ $C_1$-$C_6$-Alkyl, 1-fach mit $C_1$-$C_3$-Alkoxy oder $C_3$-$C_6$-Cycloalkyl und/oder 1-bis 3-fach mit Halogen substituiertes $C_1$-$C_6$-Alkyl, $C_3$-$C_6$-Cycloalkyl, Phenyl, 1- bis 3-fach mit Halogen und/oder $C_1$-$C_3$-Alkyl substituiertes Phenyl, Benzyl oder 1- bis 3-fach mit Halogen und/oder $C_1$-$C_3$-Alkyl substituiertes Benzyl;

$R_3$ Wasserstoff oder den Rest CO-$R_7$;

$R_4$ $C_3$-$C_6$-Cycloalkyl, 1- bis 3-fach mit Halogen und/oder Methyl substituiertes $C_3$-$C_6$-Cycloalkyl, $C_2$-$C_5$-Alkinyl, $C_1$-$C_4$-Alkylthio oder den Rest $CH_2XR_8$;

$R_5$ $C_1$-$C_6$-Alkyl, mit Hydroxy oder $C_1$-$C_3$-Alkoxy und/oder mindestens einfach mit Halogen substituiertes $C_1$-$C_6$-Alkyl, $R_5$ ferner $C_3$-$C_5$-Alkenyl, Halogen, Hydroxy, $C_1$-$C_3$-Alkoxy, $C_1$-$C_3$-Haloalkoxy, $C_3$-$C_6$-Cycloalkyl oder $C_1$-$C_3$-Alkylthio;

$R_6$ unabhängig voneinander $C_1$-$C_4$-Alkyl, $C_1$-$C_2$-Alkoxy, $C_1$-$C_2$-Haloalkoxy oder Halogen;

$R_7$ $C_1$-$C_4$-Alkyl oder mindestens einfach mit Halogen substituiertes $C_1$-$C_4$-Alkyl;

$R_8$ $C_1$-$C_6$-Alkyl oder mindestens einfach mit Halogen oder $C_1$-$C_4$-Alkoxy substituiertes $C_1$-$C_6$-Alkyl und

X Sauerstoff.

5. Verbindungen der Formel I gemäss Anspruch 1, worin bedeuten:

$R_1$ bis zu 3-fach mit $R_6$ substituiertes Phenyl mit der Massgabe, dass mindestens die ortho-Stellung im Phenylring substituiert ist;
$R_2$ $C_1$-$C_3$-Alkyl;
$R_3$ Wasserstoff;
$R_4$ $C_3$-$C_6$-Cycloalkyl, mit $CH_3$ substituiertes $C_3$-$C_6$-Cycloalkyl, $C_2$-$C_4$-Alkinyl, $C_1$-$C_3$-Alkylthio oder den Rest $CH_2X$-$R_8$;
$R_5$ $C_1$-$C_3$-Alkyl, mit Halogen substituiertes $C_1$-$C_3$-Alkyl, $R_5$ ferner $C_3$-$C_5$-Alkenyl, $C_3$-$C_5$-Cycloalkyl oder $C_1$-$C_3$-Alkylthio;
$R_6$ unabhängig voneinander $C_1$-$C_4$-Alkyl, Halogen oder $C_1$-$C_4$-Haloalkyl;
$R_8$ $C_1$-$C_3$-Alkyl und
X Sauerstoff.

6. Verbindungen der Formel I gemäss Anspruch 1, worin bedeuten:
$R_1$ $\alpha$-Naphthyl, 2-substituiertes $\alpha$-Naphthyl, 2,3-disubstituiertes oder 2,4-disubstituiertes $\alpha$-Naphthyl, wobei die Substituenten in 2- und 4-Stellung $C_1$-$C_3$-Alkyl, Halogen oder Nitro sein können und der Substituent in 3-Stellung $C_1$-$C_3$-Alkyl ist;
$R_2$ $C_1$-$C_3$-Alkyl;
$R_3$ Wasserstoff;
$R_4$ $C_3$-$C_6$-Cycloalkyl, mit $CH_3$ substituiertes $C_3$-$C_6$-Cycloalkyl, $C_2$-$C_4$-Alkinyl, $C_1$-$C_3$-Alkylthio oder den Rest $CH_2X$-$R_8$;
$R_5$ $C_1$-$C_3$-Alkyl, mit Halogen substituiertes $C_1$-$C_3$-Alkyl, $R_5$ ferner $C_3$-$C_5$-Alkenyl, $C_3$-$C_5$-Cycloalkyl oder $C_1$-$C_3$-Alkylthio;
$R_8$ $C_1$-$C_3$-Alkyl und
X Sauerstoff.

7. Verbindungen der Formel I gemäss Anspruch 6, worin bedeuten:
$R_1$ $\alpha$-Naphthyl, 2-Methyl-$\alpha$-naphthyl, 2-Methyl-4-nitro-$\alpha$-naphthyl, 2,4-Dimethyl-$\alpha$-naphthyl, 2,3-Dimethyl-$\alpha$-naphthyl oder 4-Methyl-$\alpha$-naphthyl.

8. Eine Verbindung der Formel I gemäss Anspruch 1 ausgewählt aus:
4-Methyl-6-cyclopropyl-2-[1-(2-methylphenyl)-ethylidenhydrazino]-pyrimidin;
4-Methyl-6-methoxymethyl-2-[1-(2-methylphenyl)-ethylidenhydrazino]-pyrimidin;
4-Methyl-6-cyclopropyl-2-[1-(2-chlorphenyl)-ethylidenhydrazino]-pyrimidin;
4-Methyl-6-(1-propinyl)-2-[1-(2-methylphenyl)-ethylidenhydrazino]-pyrimidin;
4-Methyl-6-(1-propinyl)-2-[1-(2-chlorphenyl)-ethylidenhydrazino]-pyrimidin;
4-Fluormethyl-6-cyclopropyl-2-[1-(2-methylphenyl)-ethylidenhydrazino]-pyrimidin;
4-Methyl-6-fluormethyl-2-[1-(2-methylphenyl)-ethylidenhydrazino]-pyrimidin;
4-Methyl-6-cyclopropyl-2-[1-(2,4-dimethylphenyl)-ethylidenhydrazino]-pyrimidin;
4-Methyl-6-cyclopropyl-2-[1-(2-methyl-$\alpha$-naphthyl)-ethylidenhydrazino]pyrimidin (Verb. Nr. 3.25);
4-Methyl-6-cyclopropyl-2-[1-(2,4-dimethyl-$\alpha$-naphthyl)-ethylidenhydrazino]-pyrimidin (Verb. Nr. 3.26);
4-Methyl-6-cyclopropyl-2-[1-(2,5-dimethylphenyl)-ethylidenhydrazino]-pyrimidin (Verb. Nr. 1.35);
4-Methyl-6-cyclopropyl-2-[1-(2-bromphenyl)-ethylidenhydrazino]-pyrimidin (Verb. Nr. 1.45);
4-Methyl-6-thiomethyl-2-[1-(2-methylphenyl)-ethylidenhydrazino]-pyrimidin (Verb. Nr. 1.168).

9. Verfahren zur Herstellung der Verbindungen der Formel I von Anspruch 1, dadurch gekennzeichnet, dass man
1) ein aromatisches Keton der Formel II

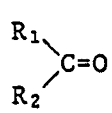

(II)

mit einem Hydrazinopyrimidin der Formel III

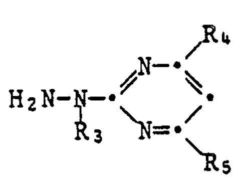

(III),

worin $R_1$-$R_5$ die unter Formel I angegebenen Bedeutungen besitzen, oder einem Salz davon in einem

inerten Lösungsmittel oder in der Schmelze bei Temperaturen von -20° bis 200° C, bevorzugt 20° C bis zum Siedepunkt des verwendeten Lösungsmittels, umsetzt, wobei das Lösungsmittel auch das Keton der Formel II sein kann, und wobei ferner die Zugabe eines Katalysators in Form einer geringen Menge einer organischen oder anorganischen Säure oder einer Base eine beträchtliche Beschleunigung der Umsetzung bewirken kann; oder

2) ein Hydrazon der Formel IV

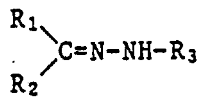

(IV)

mit einem Pyrimidin der Formel V

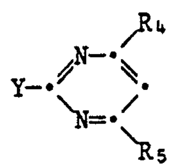

(V),

worin Y Halogen, bevorzugt Chlor oder Brom, $C_1$-$C_4$-Alkoxy, bevorzugt Methoxy oder Ethoxy, Phenoxy, Mercapto, $C_1$-$C_4$-Alkylthio, bevorzugt Methylthio oder Ethylthio, Phenylthio, $C_1$-$C_4$-Alkylsulfonyl, bevorzugt Methylsulfonyl oder Ethylsulfonyl, oder Phenylsulfonyl darstellt und $R_1$-$R_5$ die unter Formel I angegebenen Bedeutungen besitzen, in Gegenwart einer Base in einem inerten Lösungsmittel bei Temperaturen von -50° bis 150° C, bevorzugt von 20° C bis zum Siedepunkt des verwendeten Lösungsmittels, umsetzt; oder

3) ein Amidinohydrazon der Formel VI

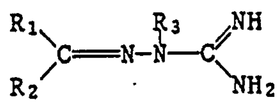

(VI)

mit einem Diketon der Formel VII

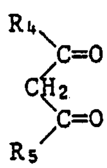

(VII),

worin $R_1$-$R_5$ die unter Formel I angegebenen Bedeutungen besitzen, in einem inerten Lösungsmittel oder in einer Schmelze bei Temperaturen von -20° bis 200° C, bevorzugt von 20° C bis zum Siedepunkt des verwendeten Lösungsmittels, umsetzt, wobei das Lösungsmittel auch das Diketon der Formel VII sein kann.

10. Verfahren zur Herstellung von Verbindungen der Formel I, in denen $R_3$ $C_1$-$C_6$-Alkyl oder CO-$R_7$ darstellt und $R_7$ $C_1$-$C_6$-Alkyl oder ein mindestens einfach mit Halogen und/oder $C_1$-$C_3$-Alkoxy substituiertes $C_1$-$C_6$-Alkyl bedeutet, indem man
eine Verbindung der Formel Ia

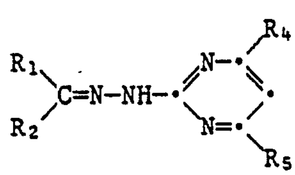

(Ia)

a) mit einer Verbindung der Formel VIII
$R_3$-Z (VIII)

worin Z eine übliche Abgangsgruppe, wie z.B. Halogen oder Sulfonyloxy, bedeutet und $R_1$, $R_2$, $R_4$ und $R_5$ die unter Formel I angegebenen Bedeutungen besitzen, in Gegenwart einer starken Säure oder ohne diese in einem inerten Lösungsmittel umsetzt; oder

b) mit einem reaktionsfähigen Derivat einer Säure der Formel

$R_7$-COOH (IX),

wobei die reaktionsfähigen Derivate der Verbindungen der Formel IX die Halogenide, bevorzugt Chloride oder Bromide, oder die Anhydride darstellen.

11. Verfahren zur Herstellung von Verbindungen der Formel Ic

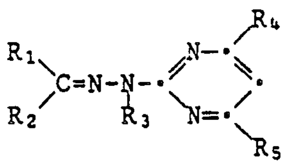

(Ic)

worin $R_5'$ Chlor oder Brom darstellt und $R_1$-$R_4$ die unter Formel I angegebenen Bedeutungen besitzen, indem man eine Verbindung der Formel Ib

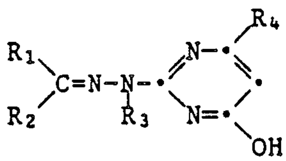

(Ib)

mit Phosphoroxychlorid oder Phosphoroxybromid in einem inerten Lösungsmittel bei Temperaturen von -20° C bis 180° C, bevorzugt von 20° C bis zum Siedepunkt des verwendeten Lösungsmittels, umsetzt, wobei als Lösungsmittel auch das betreffende Phosphoroxyhalogenid eingesetzt werden kann.

12. Verfahren zur Herstellung von Verbindungen der Formel Id

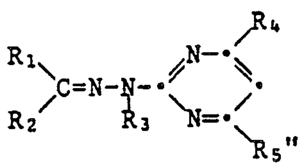

(Id)

worin $R_5''$ $C_1$-$C_3$-Alkoxy, $C_1$-$C_3$-Haloalkoxy oder $C_1$-$C_3$-Alkylthio darstellt und $R_1$-$R_4$ die unter Formel I angegebenen Bedeutungen besitzen, indem man eine Verbindung der Formel Ic

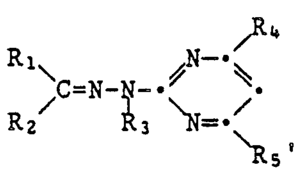

(Ic)

worin $R_5'$ Chlor oder Brom bedeutet, mit einer Verbindung der Formel XI

$R^a$-X-M (XI)

worin $R^a$ $C_1$-$C_3$-Alkyl oder $C_1$-$C_3$-Haloalkyl, X Sauerstoff oder Schwefel und M Wasserstoff oder vorzugsweise ein Metallatom, insbesondere ein Alkaliatom, bedeuten, in einem inerten Lösungsmittel bei Temperaturen von -20° bis 180° C, bevorzugt 20° bis zum Siedepunkt des verwendeten Lösungsmittels reagieren lässt, wobei im Falle, dass M Wasserstoff darstellt, die Verbindung XI auch als Lösungsmittel eingesetzt werden kann.

13. Verbindungen der Formel III

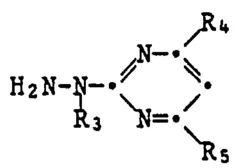

(III)

worin bedeuten:

$R_3$ Wasserstoff, $C_1$-$C_6$-Alkyl oder CO-$R_7$;

$R_4$ $C_3$-$C_6$-Cycloalkyl, bis zu 3-fach mit Halogen und/oder Methyl substituiertes $C_3$-$C_6$-Cycloalkyl, mindestens einfach mit Halogen, Hydroxy und/oder Cyano substituiertes $C_1$-$C_4$-Alkyl, $C_2$-$C_5$-Alkinyl, $C_1$-$C_4$-Alkylthio oder den Rest $CH_2XR_8$;

$R_5$ Wasserstoff, $C_1$-$C_6$-Alkyl, mindestens einfach mit Halogen, Hydroxy, Cyan oder $C_1$-$C_3$-Alkoxy substituiertes $C_1$-$C_6$-Alkyl, Halogen, $C_1$-$C_3$-Alkoxy, $C_1$-$C_3$-Haloalkoxy, $C_3$-$C_6$-Cycloalkyl, $C_3$-$C_5$-Alkenyl oder $C_1$-$C_3$-Alkylthio;

$R^8$ $C_1$-$C_6$-Alkyl, mindestens einfach mit Halogen, Hydroxy, Cyano und/oder $C_1$-$C_4$-Alkoxy substituiertes $C_1$-$C_6$-Alkyl, $C_3$-$C_6$-Alkenyl, $C_3$-$C_6$-Alkinyl oder $C_3$-$C_6$-Cycloalkyl;

X Sauerstoff oder Schwefel.

14. Mittel zur Bekämpfung oder Verhütung eines Befalls durch schädliche Mikroorganismen, dadurch gekennzeichnet, dass es als aktive Komponente mindestens eine Verbindung der Formel I gemäss Anspruch 1 enthält, zusammen mit einem geeigneten Trägermaterial.

15. Mittel gemäss Anspruch 14, dadurch gekennzeichnet, dass es als aktive Komponente mindestens eine Verbindung der Formel I gemäss Anspruch 2 enthält.

16. Mittel gemäss Anspruch 14, dadurch gekennzeichnet, dass es als aktive Komponente mindestens eine Verbindung der Formel I gemäss Anspruch 3 enthält.

17. Mittel gemäss Anspruch 14, dadurch gekennzeichnet, dass es als aktive Komponente mindestens eine Verbindung der Formel I gemäss Anspruch 4 enthält.

18. Mittel gemäss Anspruch 14, dadurch gekennzeichnet, dass es als aktive Komponente mindestens eine Verbindung der Formel I gemäss Anspruch 5 enthält.

19. Mittel gemäss Anspruch 14, dadurch gekennzeichnet, dass es als aktive Komponente mindestens eine Verbindung der Formel I gemäss Anspruch 6 enthält.

20. Mittel gemäss Anspruch 14, dadurch gekennzeichnet, dass es als aktive Komponente mindestens eine Verbindung der Formel I gemäss Anspruch 7 enthält.

21. Mittel gemäss Anspruch 14, dadurch gekennzeichnet, dass es als aktive Komponente mindestens eine Verbindung der Formel I gemäss Anspruch 8 enthält.

22. Mittel gemäss Anspruch 14, dadurch gekennzeichnet, dass es 0,1 bis 99 Gew.-% eines Wirkstoffs der Formel I, 99,9 bis 1 Gew.-% eines festen oder flüssigen Zusatzstoffes und 0 bis 25 Gew.-% eines Tensides enthält.

23. Verfahren zur Bekämpfung oder Verhütung eines Befalls von Kulturpflanzen durch phytopathogene Mikroorganismen, dadurch gekennzeichnet, dass man als Wirkstoff eine Verbindung der Formel I gemäss Anspruch 1 auf die Pflanze, auf Pflanzenteile oder ihren Standort appliziert.

24. Verfahren gemäss Anspruch 23, dadurch gekennzeichnet, dass man als Wirkstoff eine Verbindung gemäss einem der Ansprüche 2 bis 8 appliziert.

25. Verfahren gemäss Anspruch 23, dadurch gekennzeichnet, dass phytopathogene Pilze bekämpft werden.

26. Verfahren zur Herstellung eines agrochemischen Mittels gemäss Anspruch 14, dadurch gekennzeichnet, dass man mindestens eine Verbindung der Formel I gemäss Anspruch 1 mit geeigneten festen oder flüssigen Zusatzstoffen und/oder Tensiden innig vermischt.

27. Verwendung von Verbindungen der Formel I gemäss Anspruch 1 zur Bekämpfung und/oder präventiven Verhütung eines Befalls von schädlichen Mikroorganismen.

28. Verwendung gemäss Anspruch 27, dadurch gekennzeichnet, dass es sich bei den Mikroorganismen um phytopathogene Pilze handelt.

29. Verwendung gemäss Anspruch 27 gegen Pilze aus den Klassen Fungi imperfecti, Ascomyceten, Oomyceten und Basidiomyceten.

30. Verwendung gemäss Anspruch 29 gegen die Species Botrytis, Pyricularia, Plasmopara, Phytophthora, Cercospora, Rhizoctonia, Fusarium oder Helminthosporium.